# EUROPEAN PATENT APPLICATION

(11) **EP 4 818 303 A1**
(43) Date of publication of application: **07.10.2026**
(21) Application number: 25168604.4
(22) Date of filing: 04.04.2025
(51) Int. Cl.: C11D 3/382, C11D 1/72, A61K 8/92, C11D 3/50, C11D 1/62, C11D 1/835

(54) **TREATMENT COMPOSITIONS CONTAINING ROSINS AND CERTAIN NON-IONIC SURFACTANTS**

(71) Applicant: The Procter & Gamble Company, Cincinnati, OH 45202 (US)
(72) Inventor: BAGLIONI, Michele, 50019 Sesto Fiorentino (IT); BAGLIONI, Piero, 50019 Sesto Fiorentino (IT); CAMERINI, Rachel Elisabetta, 50019 Sestio Fiorentino (IT); COLLU, Mattia, 1853 Strombeek-Bever (BE); ORLANDINI, Laura, 1213 Petit-Lancy - Geneva (CH); SMETS, Johan, 1853 Strombeek-Bever (BE); TAHON, Cedric Marc, 1853 Strombeek-Bever (BE); TONELLI, Monica, 50019 Sesto Fiorentino (IT)
(74) Representative: P&G Patent Belgium UK

(57) **Abstract**

The present invention relates to a treatment composition comprising particles and an adjunct ingredient, wherein the particles comprise a rosin and one or more benefit agents, wherein the one or more benefit agents comprises a fragrance material. Said composition further comprises a non-ionic surfactant comprising an alkyl alkoxylated surfactant selected from an alkyl ethoxylated surfactant, an alkyl propoxylated surfactant or combinations thereof. Said alkyl alkoxylated surfactant comprises between 4 and 12 alkoxylate groups and has an alkyl chain of between 7 and 15 carbon atoms. Said composition is made by: mixing a rosin and one or more benefit agents to create a premix; contacting the premix with a water solution comprising a non-ionic surfactant to form a slurry; and using the slurry to produce said composition. Said composition can be used to treat fabrics: by obtaining a fabric to be washed; and contacting the fabric with a wash liquor, the wash liquor comprising said composition and water.

## Description

### FIELD OF THE INVENTION

The present disclosure relates to slurries and treatment compositions that include particles and certain non-ionic surfactants, where the particles include a rosin and one or more benefit agents. The present disclosure also relates to methods of making and using such compositions.

### BACKGROUND OF THE INVENTION

Treatment compositions, such as liquid fabric softeners, can employ various delivery systems to facilitate improved delivery of benefit agents. Particles are commonly selected as a delivery system, particularly for perfumes.

Rosins may function as a perfume delivery agent when incorporated into particles. As rosins are derived from natural and/or sustainable sources, they constitute an alternative to synthetic materials, such as synthetic polymers.

Due to their intrinsic stickiness, rosins provide an enhanced deposition efficiency and high resistance to water dilution which result in improved fabric odor and customer delight across all touchpoints.

However, rosins in the finished product may cause undesirable residues on the walls of the container. Consumers may associate this phenomenon with a loss of product or with an unstable and/or spoiled product.

There is an ongoing need for treatment compositions that include rosin-based delivery systems with improved stability, dispersibility and processability.

It is an object of the present invention to provide a rosin-based treatment composition that does not leave residues on the walls of the container, i.e. is homogeneous and stable over time, while maintaining the deposition benefits offered by the rosins.

It is another object of the present invention to provide a consumer product comprising said treatment composition.

It is another object of the present invention to provide a method of making said treatment composition.

It is another object of the present invention to provide a method of treating fabric comprising using said treatment composition.

It is another object of the present invention to provide an encapsulated perfume slurry.

### SUMMARY OF THE INVENTION

It was surprisingly found that the addition of certain non-ionic surfactants to a rosin-based treatment composition reduces the insoluble residues left by the finished product on the walls of the container, thus increasing the stability, dispersibility and processability of the composition.

In a first aspect, the present invention relates to a treatment composition comprising particles and an adjunct ingredient, wherein the particles comprise a rosin and one or more benefit agents, wherein the one or more benefit agents comprises a fragrance material, characterized in that the composition comprises a non-ionic surfactant, wherein the non-ionic surfactant comprises an alkyl alkoxylated surfactant, wherein the alkyl alkoxylated surfactant is selected from an alkyl ethoxylated surfactant, an alkyl propoxylated surfactant or combinations thereof, wherein the alkyl alkoxylated surfactant comprises an alkyl chain and an alkoxy chain, wherein the alkyl chain and the alkoxy chain are covalently linked, wherein the alkyl chain is selected from a linear alkyl chain, a branched alkyl chain, a cycloalkyl chain or mixtures thereof, wherein the alkyl chain comprises between 7 and 15 carbon atoms, wherein the alkoxy chain comprises between 4 and 12 alkoxylate groups.

In another aspect, the present invention relates to a consumer product comprising a bottle, wherein the treatment composition according to the first aspect is contained in the bottle.

In another aspect, the present invention relates to a method of making the treatment composition according to the first aspect, said process comprising the following steps: mixing a rosin and one or more benefit agents to create a premix; contacting the premix with a water solution comprising a non-ionic surfactant to form an encapsulated perfume slurry; and using the slurry to produce the treatment composition.

In another aspect, the present invention also relates to a method of treating fabrics, the method comprising the following steps: obtaining a fabric to be treated; and contacting the fabric with a wash liquor, the wash liquor comprising the treatment composition of the first aspect and water.

In another aspect, the present invention also relates to an encapsulated perfume slurry.

### DETAILED DESCRIPTION OF THE INVENTION

The present invention relates to slurries and treatment compositions comprising rosins and benefit agents, such as fragrances, as part of the same delivery particle. These treatment compositions further comprise a non-ionic surfactant having a certain carbon length and degree of alkoxylation.

The components, compositions, and processes of the present disclosure are described in more detail below.

As used herein, the articles "a" and "an" when used in a claim, are understood to mean one or more of what is claimed or described. As used herein, the terms "include," "includes," and "including" are meant to be non-limiting. The compositions of the present disclosure can comprise, consist essentially of, or consist of, the components of the present disclosure.

The terms "substantially free of" or "substantially free from" may be used herein. This means that the indicated material is at the very minimum not deliberately added to the composition to form part of it, or, preferably, is not present at analytically detectable levels. It is meant to include compositions whereby the indicated material is present only as an impurity in one of the other materials deliberately included. The indicated material may be present, if at all, at a level of less than 1%, or less than 0.1%, or less than 0.01%, or even 0%, by weight of the composition.

The slurries of the present invention, i.e. encapsulated perfume slurries, may be used to produce the treatment compositions describe hereafter by the method of making described below in the description.

The treatment composition may be a liquid treatment composition or a solid treatment composition, preferably a liquid treatment composition. More preferably, the treatment composition is a liquid fabric care treatment composition, a liquid hard surface treatment composition, a liquid personal care treatment composition, most preferably a liquid fabric care treatment composition.

The compositions of the present disclosure may be selected from the group of light duty liquid detergents compositions, heavy duty liquid detergent compositions, detergent gels commonly used for laundry, bleaching compositions, laundry additives, fabric enhancer compositions, hard surface cleaners and mixtures thereof.

The composition may be in any suitable form. The composition may be in the form of a liquid composition, a granular composition, a single-compartment pouch, a multi-compartment pouch, a sheet, a pastille or bead, a fibrous article, a tablet, a bar, flake, or a mixture thereof. The composition can be selected from a liquid, solid, or combination thereof.

The composition can be an aqueous liquid laundry detergent composition. For such aqueous liquid laundry detergent compositions, the water content can be present at a level of from 5.0 %to 95 %, preferably from 25 %to 90 %, more preferably from 50 % to 85 % by weight of the liquid detergent composition.

The pH range of the detergent composition is from 6.0 to 8.9, preferably from pH 7 to 8.8.

The detergent composition can also be encapsulated in a water-soluble film, to form a unit dose article. Such unit dose articles comprise a detergent composition of the present invention, wherein the detergent composition comprises less than 20%, preferably less than 15%, more preferably less than 10% by weight of water, and the detergent composition is enclosed in a water-soluble or dispersible film. Such unit-dose articles can be formed using any means known in the art. Suitable unit-dose articles can comprise one compartment, wherein the compartment comprises the liquid laundry detergent composition. Alternatively, the unit-dose articles can be multi-compartment unit-dose articles, wherein at least one compartment comprises the liquid laundry detergent composition.

The treatment composition may be a solid treatment composition. The skilled person is aware of the composition and properties of solid treatment compositions. The solid treatment composition may be a granular composition comprising a plurality of granules. The granules may be spray dried, agglomerated, extruded or a mixture thereof. Typically, the granular laundry detergent composition is a fully formulated laundry detergent composition, not a portion thereof such as a spray-dried, extruded or agglomerate granule that only forms part of the laundry detergent composition.

The laundry treatment composition may be in the form of a water-soluble porous flexible solid sheet. The water-soluble porous flexible solid sheet may comprise a water-soluble polymer, a surfactant.

The laundry treatment composition may be in the form of a fabric enhancer bead. Said fabric enhancer beads may comprise a water-soluble carrier. The water-soluble carrier acts to carry the delivery particle to the wash liquor. Upon dissolution of the water-soluble carrier, the delivery particle is dispersed into the wash liquor and deposited onto the laundry.

The water-soluble carrier can be a material that is soluble in a wash liquor within a short period of time, for instance less than about 10 minutes.

The laundry treatment composition may be in the form of a solid dissolvable composition. Solid dissolvable compositions (SDC) comprise crystallizing agent, water, and an active agent. The crystallizing agent primarily consists of sodium salt of saturated fatty acids having from 8 to 12 methylene groups, where the sodium counter ion enables formation of fiber crystal mesh (counter ions other than sodium result in non-fiber crystals) in the composition.

The treatment composition may be a liquid hard surface treatment composition. The skilled person is aware of the composition and properties of liquid hard surface treatment compositions.

The liquid hard surface composition can be an aqueous cleaning composition. The cleaning composition can comprise more than 50%, preferably more than 60% by weight of the composition of water. The composition may comprise from 80% to 99.5%, optionally from 85% to 99%, optionally from 94% to 98%, optionally from 95% to 99.93%, optionally from 97% to 99.93%, optionally from 98% to 99.9%, optionally from 98% to 99.5%, optionally from 98% to 99%, water by weight of the cleaning composition.

The cleaning composition can comprise from 0.05% to 4% perfume by weight of the cleaning composition. Perfume includes aromatic compounds, including but not limited to, essential oils and synthetic fragrance ingredients and mixtures thereof.

The cleaning composition can comprise 0.02% to 10%, optionally from 0.04% to 5%, optionally from 0.1% to 4%, optionally from about 0.5% to about 2%, and any ranges within any of the aforesaid ranges to two decimal places, nonionic surfactant by weight of the cleaning composition. The nonionic surfactant can be an ethoxylated non-ionic surfactant, an alkyl polyglucoside, and mixtures thereof.

The cleaning composition may comprise up to 5% by weight, optionally up to 3%, optionally up to 1%, of an additional surfactant, preferably selected from: anionic, amphoteric, zwitterionic, and mixtures thereof. The zwitterionic surfactant may comprise an amine oxide.

The cleaning composition can comprise a solvent system comprising C₁-C₄ alcohols, glycol ether solvents, and mixtures thereof.

The cleaning composition can have a pH from about 2 to about 11, optionally from about 2 to about 5, optionally from about 6 to about 9, optionally from about 8 to about 11.

The treatment composition may be a liquid personal care treatment composition. The skilled person is aware of the composition and properties of liquid personal care treatment compositions. The liquid personal care composition may comprise greater than about 1% by weight of a surfactant system which provides cleaning performance to the composition or may comprise greater than 5% by weight of a surfactant system which enables solubilization of a scalp care active. In addition, the composition may have sufficient surfactant to enable micellar or polymeric thickening. The surfactant system comprises an anionic surfactant and/or a combination of anionic surfactants and/or a combination of anionic surfactants and co-surfactants selected from the group consisting of amphoteric, zwitterionic, nonionic and mixtures thereof.

The personal care composition may comprise from about 10% to about 23%, from about 12% to about 21%, from about 10% to about 18%, by weight of one or more surfactants.

Anionic surfactants suitable for use in the compositions are the alkyl, alkyl ether sulfates, anionic alkyl and alkyl ether sulfosuccinates and/or dialkyl and dialkyl ether sulfosuccinates and mixtures thereof. The personal care composition may comprise a co-surfactant. The co-surfactant can be selected from the group consisting of amphoteric surfactant, zwitterionic surfactant, non-ionic surfactant and mixtures thereof. The co-surfactant can include, but is not limited to, lauramidopropyl betaine, cocoamidopropyl betaine, lauryl hydroxysultaine, sodium lauroamphoacetate, disodium cocoamphodiacetate, cocamide monoethanolamide, alkyl glycosides, alkyl polyglucosides, glyceril ester, polyglyceryl esters, sorbitan esters, alkylphenol ethoxylates, and mixtures thereof. The personal care composition may further comprise from about 0.5% to about 8%, from about 1.0% to about 7%, from about 1.5% to about 6%, by weight of one or more amphoteric, zwitterionic, nonionic co-surfactants, or a mixture thereof.

The present composition may comprise a humectant. The composition contains a safe and effective amount of the humectant. In particular, it may contain from about 20% to about 70%; from about 20% to about 50%; from about 23% to about 45%; by weight of a humectant. The composition may contain two or more different humectants, for example, the composition may contain glycerin and xylitol.

The personal care composition may comprise a thickening polymer to increase the viscosity of the composition. Suitable thickening polymers can be used. The personal care composition may comprise from about 0.05% to about 10% of a thickening polymer, from about 0.05% to about 5% of a thickening polymer, from about 0.05%to about 2.5%% of a thickening polymer, and from about 0.05%% to about 2% of a thickening polymer. The thickening polymer modifier may be a polyacrylate, polyacrylamide thickeners. The thickening polymer may be an anionic thickening polymer. The thickening polymers may be combined with cellulose and derivatives, with a guar and guar derivatives, with polyethylene oxide:polypropylene oxide; and POE-PPO copolymers.

The present composition may comprise a scalp care active. The scalp care active includes soluble scalp care actives and scalp health agents.

The treatment composition may be a conditioning composition, such as a liquid fabric enhancer composition or a hair conditioner composition.

Most preferably, the treatment composition may be a liquid fabric care treatment composition. The liquid fabric care treatment composition can be Newtonian or non-Newtonian, preferably non-Newtonian. The liquid fabric care treatment composition can comprise from 5% to 99%, or from 15% to 90%, or from 25% to 80% by weight of the liquid fabric care composition of water.

As used herein the phrase "fabric care composition" includes compositions and formulations designed for treating fabric. Such compositions include but are not limited to, laundry cleaning compositions and detergents, fabric softening compositions, fabric enhancing compositions, fabric freshening compositions, laundry prewash, laundry pretreat, laundry additives, spray products, dry cleaning agent or composition, laundry rinse additive, wash additive, post-rinse fabric treatment, ironing aid, unit dose formulation, delayed delivery formulation, detergent contained on or in a porous substrate or nonwoven sheet, and other suitable forms that may be apparent to one skilled in the art in view of the teachings herein. Such compositions may be used as a pre-laundering treatment, a post-laundering treatment, or may be added during the rinse or wash cycle of the laundering operation.

Unless otherwise noted, all component or composition levels are in reference to the active portion of that component or composition, and are exclusive of impurities, for example, residual solvents or by-products, which may be present in commercially available sources of such components or compositions.

All temperatures herein are in degrees Celsius (°C) unless otherwise indicated. Unless otherwise specified, all measurements herein are conducted at 20°C and under the atmospheric pressure.

In all aspects of the present disclosure, all percentages are by weight of the total composition, unless specifically stated otherwise. All ratios are weight ratios, unless specifically stated otherwise.

It should be understood that every maximum numerical limitation given throughout this specification includes every lower numerical limitation, as if such lower numerical limitations were expressly written herein. Every minimum numerical limitation given throughout this specification will include every higher numerical limitation, as if such higher numerical limitations were expressly written herein. Every numerical range given throughout this specification will include every narrower numerical range that falls within such broader numerical range, as if such narrower numerical ranges were all expressly written herein.

### Treatment composition

The present disclosure relates to treatment compositions that comprise particles, an adjunct ingredient and a non-ionic surfactant.

The treatment composition may be encapsulated in a water-soluble film, so as to be in the form of a unitized unit dose article, such as a pouch. The water-soluble film may be a polyvinyl alcohol water-soluble film. Suitable films are available from MonoSol, LLC (Indiana, USA). The treatment composition can be encapsulated in a single or multi-compartment pouch. A multi-compartment pouch may have at least two, at least three, or at least four compartments. A multi-compartmented pouch may include compartments that are side-by-side and/or superposed. The composition contained in the pouch or compartments thereof may be liquid, solid (such as powders), or combinations thereof; in such cases, at least one encapsulated composition is a liquid composition.

The treatment composition may be in the form of a unit dose article, such as a pouch or a sachet, the composition may be encapsulated by a water-soluble film. A water-soluble unit dose article may comprise at least one water-soluble film shaped such that the unit dose article comprises at least one internal compartment surrounded by the water-soluble film.

The at least one compartment comprises the treatment composition. The unit dose article may comprise more than one compartment, even at least two compartments, or even at least three compartments, or even at least four compartments, or even at least five compartments. The compartments may be arranged in superposed orientation, i.e. one positioned on top of the other. Alternatively, the compartments may be positioned in a side-by-side orientation, i.e. one orientated next to the other. The compartments may even be orientated in a "tire and rim" arrangement, i.e. a first compartment is positioned next to a second compartment, but the first compartment at least partially surrounds the second compartment but does not completely enclose the second compartment. Alternatively, one compartment may be completely enclosed within another compartment. When one compartment comprises a treatment composition according to the present disclosure, another compartment may comprise a solid, a liquid, or a mixture thereof.

The film of the present invention may be soluble or dispersible in water (e.g., at 20°C). Preferred film materials include polymeric materials. The film material can, for example, be obtained by casting, blow-moulding, extrusion or blown extrusion of the polymeric material, as known in the art. Preferably, the water-soluble film comprises polyvinyl alcohol polymer or copolymer, preferably a blend of polyvinylalcohol polymers and/or polyvinylalcohol copolymers, preferably selected from sulphonated and carboxylated anionic polyvinylalcohol copolymers especially carboxylated anionic polyvinylalcohol copolymers, most preferably a blend of a polyvinylalcohol homopolymer and a carboxylated anionic polyvinylalcohol copolymer. Suitable films include those supplied by MonoSol, LLC (Indiana) under the trade references M8630, M8900, M8779, and/or M8310. The film may comprise an aversive agent, for example a bittering agent. Prior to be being formed into a unit dose article, the water-soluble film preferably has a thickness of from 20 to 150 microns, preferably 35 to 125 microns, even more preferably 50 to 110 microns, most preferably 76 microns.

The treatment composition may be in the form of a liquid. The liquid composition may include from 30%, or from 40%, or from 50%, to 99%, or to 95%, or to 90%, or to 75%, or to 70%, or to 60%, by weight of the composition, of water. The liquid composition may be a liquid laundry detergent, a liquid fabric conditioner, a liquid dish detergent, a hair shampoo, a hair conditioner, or a mixture thereof.

The composition may be characterized by a viscosity. The composition may have a viscosity of from 1 to 1500 centipoises (1-1500 mPa*s), from 50 to 5 1000 centipoises (50-1000 mPa*s), or from 100 to 500 centipoises (100-500 mPa*s), or from 100 to 200 centipoises (100-200 mPa*s), at 20 s-1 and 21°C, is disclosed. Relatively lower viscosities allow for improved dosing and/or less residue in a dispenser drawer. Viscosity is determined according to the method provided in the Test Methods section below.

The treatment compositions of the present disclosure may be characterized by a pH of from 2 to 12, or from 2 to 8.5, or from 2 to 7, or from 2 to 5. The treatment compositions of the present disclosure may have a pH of from 2 to 4, preferably a pH of from 2 to 3.7, more preferably a pH from 2 to 3.5, preferably in the form of an aqueous liquid. It is believed that such pH levels facilitate the stability of certain adjuncts, such as conditioning actives (e.g., esterquats). The pH of a composition is determined by dissolving/dispersing the composition in deionized water to form a solution at 10% concentration, at 20°C.

In a first aspect, the present invention relates to a treatment composition comprising particles and an adjunct ingredient, wherein the particles comprise a rosin and one or more benefit agents, wherein the one or more benefit agents comprises a fragrance material, **characterized in that** the composition comprises a non-ionic surfactant, wherein the non-ionic surfactant comprises an alkyl alkoxylated surfactant, wherein the alkyl alkoxylated surfactant is selected from an alkyl ethoxylated surfactant, an alkyl propoxylated surfactant or combinations thereof, wherein the alkyl alkoxylated surfactant comprises an alkyl chain and an alkoxy chain, wherein the alkyl chain and the alkoxy chain are covalently linked, wherein the alkyl chain is selected from a linear alkyl chain, a branched alkyl chain, a cycloalkyl chain or mixtures thereof, wherein the alkyl chain comprises between 7 and 15 carbon atoms, wherein the alkoxy chain comprises between 4 and 12 alkoxylate groups.

Alternatively, the first aspect may relate to a fabric care treatment composition, preferably a liquid fabric care treatment composition.

When the rosin and the benefit agents, i.e. fragrances, are mixed together, they form encapsulates and/or particles spontaneously. The properties of these particles are described later in the description. The composition also comprises an adjunct ingredient which will also be described later in the description. Rosins have high resistance to dilution and optimal deposition on fabrics which make them optimal delivery agents for fragrances. However, treatment compositions containing rosins often have an increased stickiness towards the material of commonly-used containers, thus leaving undesirable and non-resuspendable residues and/or pellets. Surprisingly, certain non-ionic surfactants with the characteristics described hereafter, reduce the buildup of residues on the walls of containers.

Without wishing to be bound by theory, the non-ionic alkoxylated surfactants disclosed hereafter have an optimal emulsifying effect on the rosin-based particles, reducing the oil-water phase separation and therefore improving the stability and resuspendability of the finished product. By preventing the formation of residues on the walls of the container, the consumer is able to recover and use the whole product.

The containers and their internal walls may be made from, but are not limited to, plastic (e.g. PET), glass or combinations thereof.

Furthermore, non-ionic surfactants according to the present invention lead to a stabilization of the particles. The stability of the particles may be assessed by optical microscopy according to the Test Method described below.

Without wishing to be bound by theory, the particle stabilization is connected with the reduced oil-water phase separation. The non-ionic surfactant shields the rosin from having excessive hydrophobic interactions with the walls of the container and with the treated fabrics, thus preventing the formation of insoluble or detergent-resistant stains thereon.

Additionally, it was surprisingly found that the treatment compositions retained the same level of particle deposition, i.e. fragrance deposition, onto the fabric. It is believed that the non-ionic surfactant does not interfere with the interaction and affinity between the particles and the fabric. In other words, the intrinsic stickiness of the rosin towards the fabric is maintained, whereas it is selectively diminished against the container's material.

Upon fabric treatment, the deposited rosin, in the form of particles, release the fragrance throughout washing/drying steps and during fabric use by the consumer. It is believed that the consumer might want to remove completely the deposited rosin in the following laundry after the treated fabric use to avoid build up and the rise of macroscopic rosin stains. The removal of these stains may be difficult to achieve, even using high-active detergents, because of the strong association between the rosin and the fabric.

It was surprisingly found that, rosin deposited on fabrics by treatment compositions according to the present invention are more easily cleanable compared to the same fabrics stained by treatment compositions lacking non-ionic surfactants. It is believed that the presence of the non-ionic surfactants, possibly associated to the rosin on the fabric, allows an easier removal of the deposited rosin when the fabric is washed with water and suitable detergents.

The skilled person is aware of suitable detergents that would be able to remove deposited rosin from fabrics. Surfactant systems as described below may be suitable for this purpose.

### Non-ionic surfactants

Non-ionic surfactants of the present invention comprise an alkyl alkoxylated surfactant, wherein the alkyl alkoxylated surfactant is selected from an alkyl ethoxylated surfactant, an alkyl propoxylated surfactant or combinations thereof. The skilled person is aware of the chemical structure of these surfactants. By reading this, the skilled person will be aware that the present definition of non-ionic surfactants includes the following:
the alkyl alkoxylated surfactant may be a mixture of single molecules wherein each molecule is either an alkyl ethoxylated surfactant or an alkyl propoxylated surfactant and/or wherein each molecule comprises an alkoxy chain comprising any combination of ethoxylate and/or propoxylate groups and/or wherein each molecule comprises an alkoxy chain comprising any combination of alkoxylate groups interspaced by one or more atoms.

The alkyl alkoxylated surfactant comprises an alkyl chain and an alkoxy chain, wherein the alkyl chain and the alkoxy chain are covalently linked.

The alkyl chain is selected from a linear alkyl chain, a branched alkyl chain, a cycloalkyl chain or mixtures thereof, preferably a linear alkyl chain.

Suitable branched alkyl alkoxylated surfactants may comprise branched alkyl chains, wherein the branched alkyl chains may comprise between 10% and 50%, preferably between 15% and 40%, of branching.

Suitable cycloalkyl alkoxylated surfactants may comprise cycloalkyl chains, wherein the cycloalkyl chain may comprise one or more ring structures selected from cicloalkanes, cycloalkenes, benzene rings, derivatives thereof or mixtures thereof.

Each ring structure may comprise between 5 and 7 carbon atoms, or even between 5 and 6 carbon atoms, that form the ring structure.

In a preferred aspect of the present invention, the non-ionic surfactant may have a hydrophilic-lipophilic balance between 10 and 15, more preferably between 11.5 and 13.9, or even between more than 12.5 and 13.9. The skilled person is aware of how to measure the hydrophilic-lipophilic balance of a substance. An exemplified version is shown in the Test Methods of the present patent.

In a preferred aspect of the present invention, the alkyl alkoxylated surfactant may be an alkyl ethoxylated surfactant, the alkoxy chain may be an ethoxy chain and the alkoxylate groups may be ethoxylate groups. The skilled person is aware of the common methods to obtain an ethoxylated surfactant. A list of suitable commercially available ethoxylated surfactants is the following: Exaal 1315 (EXXONMOBIL), Lutensol XL60 (BASF), Lutensol TO7 (BASF), Marlipal 1216/7 (SASOL), Marlipal 26 E9 (SASOL), Neodol 91-6 (SHELL), Neodol 45-7 NI (SHELL), Tergitol 15s-9 (DOW), BT5 (NIKKOL), BT7 (NIKKOL), BT9 (NIKKOL), BT12 (NIKKOL), BL9 (NIKKOL).

The alkyl alkoxylated surfactant may be an alkyl propoxylated surfactant, the alkoxy chain may be a propoxy chain and the alkoxylate groups may be propoxylate groups.

Propoxylation has similar chemical characteristics of ethoxylation. It is believed that a fully or partially propoxylated surfactant may represent a suitable alkyl alkoxylated surfactant.

A non-limiting example of a suitable commercially available propoxylated surfactants is EcosurfTM EH 9 (DOW).

In a preferred aspect of the present invention, the alkyl chain may comprise between 7 and 15 carbon atoms, preferably between 12 and 14 carbon atoms. The alkoxy chain may comprise between 4 and 12 alkoxylate groups, preferably between 7 and 9 alkoxylate groups.

In one aspect of the present invention, the non-ionic surfactant may be present between more than 0% and 10%, or even between more than 0% and 5%, or even between 0.4% and 2.5%, preferably between 0.4% and 1.8%, by weight of the composition, or the weight ratio between the rosin and the non-ionic surfactant may be 1:4 to 4:1, or even 1:1.6 to 2.4:1, preferably 1:1.2 to 1.3:1, more preferably 1:1.2.

Without wishing to be bound by theory, a non-ionic ethoxylated surfactant having an alkyl chain of 12 to 14 carbon atoms and an ethoxy chain of 7 to 9 ethoxylate groups presents the optimal chemico-physical properties to act as an emulsifier to the rosin-based particles leading to the technical benefits. This is particularly so, when the non-ionic surfactant is between 0.4% and 1.8% by weight of the composition or when the weight ratio between the rosin and the non-ionic surfactant is 1:1 to 1.35:1 or a mixture thereof.

### Particles

The present disclosure relates to particles, which are intended as "delivery particles". The particles comprise a rosin and one or more benefit agent. The particles may be hard capsules, soft capsules or mixtures thereof. The particles may form a suspension or an emulsion in the water environment. When the particles are hard capsules, a suspension is formed. A suspension is considered a heterogeneous mixture in which solid particles are dispersed throughout a liquid or gas but are not dissolved. When the particles are soft capsules, an emulsion is formed. An emulsion is considered a type of colloidal mixture in which two immiscible liquids, typically oil and water, are combined with the help of an emulsifying agent. The composition may preferably be characterized by an emulsion of soft capsules inside a water environment.

Without wishing to be bound by theory, the benefit agent alone would form immiscible and unstable droplets into the water environment. When solid rosins are heated and mixed with the benefit agents, soft capsules form, where the resin functions as an emulsifying agent, thus creating a stable emulsion inside the water environment.

The one or more benefit agents may be encapsulated in the rosin, and/or embedded in the rosin. Compositions of the present disclosure may comprise the presently described particles.

The particles of the present disclosure may be present in a population, which may have a volume-weighted average diameter (or "diameter" as used herein). Volume-weighted average diameter is determined according to the method provided in the Test Method section below.

The particles may be characterized by a volume-weighted median particle size between more than 0 um and 400 um, or between more than 0 um and 300 um, or between more than 0 um and 250 um, or between more than 0 um and 200 um, or between 0 um and 150 um, or between 0 um and 125 um, preferably between more than 0 um and 100 um, more preferably between more than 0 um and 90 um.

In a preferred aspect of the present invention, the particles may be characterized by a volume-weighted median particle size between 0.5 um and 40 um, preferably between 1 um and 30 um.

We surprisingly found that the particles according to the present invention were generally smaller and more stable compared to a composition without the non-ionic surfactant. It is believed that the non-ionic surfactant is able to stabilize relatively small particles, wherein the particles' size corresponds to an optimal compromise between good particle deposition properties and reduced stickiness to the container's material.

One or more particles of the present disclosure may comprise at least one region comprising a benefit agent, such as a perfume raw material or enzyme. The region may comprise a benefit agent, such as fragrance, being encompassed or encapsulated within the rosin.

One or more particles of the present disclosure may have a structure selected from the group consisting of: (a) a particle comprising a single region having benefit agent that is embedded in the rosin; (b) a particle comprising at least two regions having benefit agents that are embedded in the rosin; (c) a particle comprising at least one region having benefit agents that is at least partially embedded on the surface of the rosin; (d) a particle comprising a single region having a benefit agent that is embedded in the rosin and at least one region having a benefit agent that is at least partially embedded on the surface of the rosin; and (e) a particle comprising at least two regions having benefit agents that are embedded in the rosin and at least one region having a benefit agent that is at least partially embedded on the surface of the rosin.

Compositions of the present disclosure may include one or more particles having a structure according to (a)-(e), or mixtures thereof.

The particles may be characterized by a weight ratio of the rosin to the benefit agent (e.g., fragrance).

In a preferred aspect of the present invention, the weight ratio between the rosin and the fragrance material in the composition may be 1:4 to 4:1, preferably 1:3.5 to 2:1, more preferably 1:3.5 to 1:1, most preferably 1:3.

Weight ratios of rosin to benefit agent closer to 1:3 may be preferred to deliver the primary benefit, i.e. reduced stickiness to the walls of the container. When the weight ratio of rosin to benefit agent is closer to 1:1, the non-ionic surfactant surprisingly provided an additional benefit in the form of reduced residue buildup on a stainless steel material. As stainless steel is often used in machineries in manufacturing plans, it is believed that the present formulation might deliver a processability benefit compared to current rosin-based treatment composition when said weight ratio of 1:1 is sought.

The compositions of the present disclosure may comprise between 0.01% and 10%, or between 0.05% and 7%, or between 0.1% and 5%, more preferably between 0.8 % and 4 %, or between 1% and 3%, by weight of the composition, of the particles. The benefit agents of the particles are described in more detail below.

### Rosins

The compositions of the present disclosure relate to rosins. It is believed that the rosins will interact with the fragrance materials, as disclosed herein, to form an effective perfume delivery system. In particular, rosins provide performance benefits, for example by facilitating improved deposition and/or stability of benefit agents.

Rosins may be present from 0.01% to 10%, or from 0.01% to 5%, or from 0.05% to 3%, or from 0.1% to 1%, by weight of the treatment composition. Adding too little may result in little to no added benefit, while adding too much may result in processing challenges.

As defined herein, a rosin is a plant rosin acid, a derivative of said plant rosin acid (hereafter called rosin acid derivative), or a mixture thereof. Plant rosin acids are naturally-derived, i.e. extracted from plants. Plant rosin acids are monocarboxylic acids with a diterpenic structure. Diterpenes are a class of terpenes composed of four isoprene units, often with the molecular formula C₂₀H₃₂. Non-limited examples of plant rosin acids are shown in Table A. Plant rosin acids are further described below.

Rosin acid derivatives are the results of modifications of plant rosin acids and are therefore considered synthetic compounds. Rosin acid derivatives may be selected from, but are not limited to, rosin esters, rosin oligomers, hydrogenated rosin acids, or mixtures thereof. Preferably, rosin acid derivatives are rosin esters. Rosin esters and other rosin acid derivatives are further described below.

The rosins may be characterized by the parameters outlined below. These parameters may be represented as ranges rather than definite values. Without wishing to be bound by theory, when the rosin is selected from rosin acid derivatives, the rosin acid derivatives, e.g. a rosin ester, may be constituted by different isomers of a plant rosin acid or may have different degree of esterification. These phenomena result in a rosin having parameters that can slightly vary but always falling within a certain range that is characteristic of that specific rosin.

Rosins may be characterized by a softening point. Rosins are typically solid at room temperature, but the softening point is a measure of the glass transition temperature associated with these materials. The softening point of a rosin is determined according to method provided in the Test Method section below.

Without wishing to be bound by theory, the rosins according to the present invention are solid at room temperature, wherein room temperature is intended to be between 20°C and 25°C.

The rosin may be characterized by a softening point of from 50°C to 175°C, or from 60°C to 150°C, or from 75°C to 125°C. Rosins may need to be softened by heating in order to be incorporated into consumer products. Thus, for ease of processing and/or energy savings, rosin having relatively lower softening points (e.g., less than 125°C) may be preferred for the compositions and processes of the present disclosure. Lower softening points may also have an effect on improving the deposition aid performance of the rosin.

Rosins may be characterized by an acid number (sometimes called "acid value"). The acid number of a rosin relates to the total free acid content of these products. The acid number of a rosin is determined according to method provided in the Test Method section below.

Rosins may be characterized by an acid number less than 175, e.g., from 0 to 175. For the particles, compositions, and processes of the present disclosure, it may be preferred to use rosins having a relatively low acid number, such as less than 125, preferably less than 100, more preferably less than 75, even more preferably less than 50, more preferably less than 25, so as to have minimal effect on the final pH of the treatment composition.

The color of the rosin may be graded based on the Gardner Color standard number, ranging 1 to 18. So as to have minimal effect on the final color of the treatment composition, preferred rosins of the present disclosure may have a color grade of from 1 to 10, preferably from 1 to 8. The color grade of a rosin is determined according to method provided in the Test Method section below.

Rosins are typically relatively insoluble in water. For example, rosins according to the present disclosure may be characterized by a solubility of less than 1 g/L, or less than 100g/L, or less than 1g/L, or less than 0.1g/L, or less than 0.01g/L, in deionized water at 22°C. Without wishing to be bound by theory, it is believed that the relatively insoluble nature of the rosin of the present disclosure contribute to the deposition efficiency and performance of the associated benefit agent.

Rosins may be characterized by a density. Typically, the rosins are characterized by a density of at more than 1.0 kg/dm3, preferably at least 1.1 kg/dm3, at 25°C.

Rosins are typically flammable. For the particles, compositions, and processes of the present disclosure, it may be preferred to use rosins that have a relatively high flash point, e.g., higher than 190°C, to facilitate easier and safer processing. The flash point of a rosin is determined according to method provided in the Test Method section below.

The parameters above are also applicable and measurable for plant rosin acids and rosin acid derivatives. Plant rosin acids and rosin acid derivatives are discussed in more detail below.

### a) Plant rosin acids

Plant rosin acids are typically derived from conifer plants (class: Pinopsida), usually from pine trees (genus: Pinus). Plant rosin acids are obtained from the plant's oleo-resin, which is distilled to remove volatile terpenes, and the solid material left behind are the plant rosin acids. The solid plant rosin acids may be relatively translucent and/or glass-like. The rosin may have a color ranging, for example from faint yellow to a darker brown color, or even black.

Plant rosin acids are monocarboxylic acids having three fused rings. Plant rosin acids may be tricyclic diterpene monocarboxylic acids, for example with a molecular formula of C19H29COOH.

A plant rosin acid may be an abietic acid, a pimaric acid, a plicatic acid, or a mixture thereof. Abietic acid may comprise different isomers such as abietic acid, neoabietic acid, dehydroabietic acid, palustric acid, levopimaric acid, or a mixture thereof.

Pimaric acid may comprise different isomers such as pimaric acid, isopimaric acid, sandaracopimaric acid, or mixtures thereof.

Structures of these illustrative plant rosin acids are provided below in Table A.

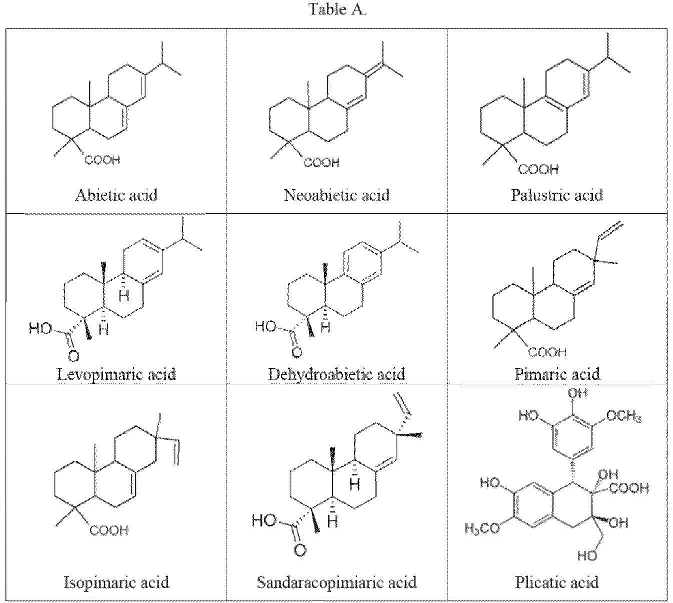

Preferably, the plant rosin acid is an abietic acid. Abietic acid has the empirical formula C19H29COOH and is also known as abietinic acid or sylvic acid.

Plant rosin acids may be classified depending on the source where they are obtained. For example, plant rosin acids may be classified as gum plant rosin acid, wood plant rosin acid, tall oil plant rosin acid, or a mixture thereof. Gum plant rosin acid may be derived from a resin extrudate of a tree or other plant and may be harvested by tapping or wounding the tree and then collecting and processing the extrudate. Wood plant rosin acid may be derived from materials that are harvested from pine tree stumps, for example through solvent extraction and/or distillation. Tall oil plant rosin acid is a byproduct of the distillation of crude tall oil during the Kraft process of wood pulp manufacture when pulping pine trees.

Suitable plant rosin acids may be obtained, for example, from a variety of pine species, such as Pinus massoniana (Masson's pine), P. elliotti (slash pine), P. palustris (longleaf pine), P. taeda (loblolly pine), P. oocarpa (Mexican yellow pine), P. leiophylla (Chihuahua pine), P. devoniana (pino lacio, or Michoacan pine), P. montezumae (Montezuma pine), P. pinaster (maritime pine), P. sylvestris (Scots pine), P. halepensis (Aleppo pine), P. insularis (Benguet pine), P. kesiya (Khasi pine), P. strobus (Eastern white pine), or mixtures thereof.

The plant rosin acids may be selected from gum plant rosin acid, wood plant rosin acid, tall oil plant rosin acid, or mixtures thereof; preferably gum plant rosin acids.

### b) Rosin esters

The rosin may preferably be a rosin ester. Rosin esters are a type of rosin acid derivatives. Rosin esters may be made by chemically modifying a plant rosin acid, through an esterification reaction. The skilled person is aware of an esterification reaction between an acid and an alcohol. A rosin ester is the reaction product of a plant rosin acid and an alcohol, as exemplified below.

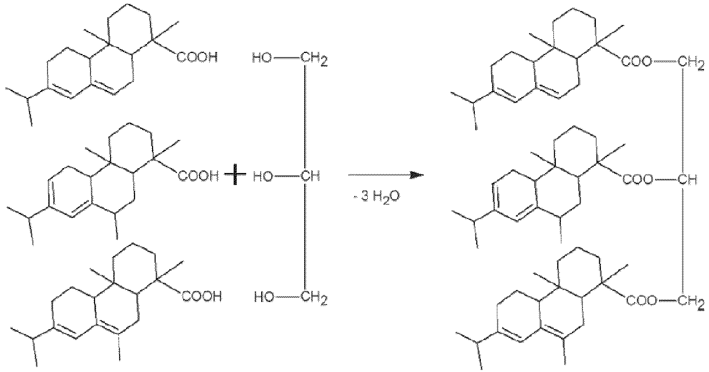

In the specific example shown, the rosin ester is formerd by a condensation reaction (i.e. esterification) between three abietic acid molecules and one glycerol molecule. A rosin ester formed by an alcohol and one or more abietic acid molecules will be hereafter called abietic rosin ester.

The rosin ester may be the ester of an alcohol and of a plant rosin acid selected from an abietic acid, a pimaric acid, plicatic acid, or mixtures therof, preferably an abietic acid. It is therefore preferrable that the rosin ester is an abietic rosin ester.

The rosin ester may be the ester of an alcohol and of a plant rosin acid selected from a gum plant rosin acid, a wood plant rosin acid, a tall oil plant rosin acid, or mixtures thereof, preferably, a gum plant rosin acid. It is therefore preferrable that the rosin ester is a gum rosin ester.

The alcohol in the esterification reaction may be a mono-alcohol, a diol, or a polyol. Suitable monoalcohols include methanol, which when reacted with a plant rosin acid can form a rosin methyl ester. The alcohol may be a polyol that comprises three or more hydroxyl groups. Suitable polyols may include a total of three hydroxyl groups (e.g., glycerol), a total of four hydroxyl groups (e.g., pentaerythritol), or a total of six hydroxyl groups (e.g., sorbitol or mannitol). Preferred polyols include glycerol, pentaerythritol, and mixtures thereof.

The rosin ester may be an ester of a plant rosin acid and an alcohol, where the alcohol comprises: (a) from one to six carbon atoms, and/or (b) from one to six hydroxyl groups. Preferably, the alcohol will comprise at least one hydroxyl group and at least one carbon atom. The alcohol from which the rosin ester is derived may be selected from the group consisting of methanol, glycerol, pentaerythritol, and mixtures thereof, preferably glycerol. Thus, the rosin ester may be a methyl ester, a glyceryl rosin ester, a pentaerythrityl rosin ester, or a mixture thereof. The rosin may include a certain amount of unreacted alcohol, preferably the rosin is substantially free from unreacted alcohol.

The rosin ester may comprise, on average, from two to six moles of ester groups per mole of rosin ester.

### C. Other Rosin Acid Derivatives

Rosin acid derivatives may be produced by subjecting plant rosin acids to processes such as hydrogenation, dimerization, polymerization, saponification, or mixtures thereof. Rosin esters are considered rosin acid derivatives.

The same rosin acid derivatives may be further derivatized. In some cases, the rosin esters may be further derivatized.

The rosin acid derivative may be a hydrogenated rosin acid. Many plant rosin acids are unsaturated, they tend to be oxidatively unstable and may undergo color changes upon storage. Hydrogenation can help to stabilize the plant rosin acids and reduce undesirable color change. Furthermore, hydrogenated rosin acids tend to have lighter colors than the parent rosin, providing more formulation and aesthetic flexibility. Hydrogenated rosin acids may be partially or fully hydrogenated. Below is a sample reaction for the partial and full hydrogenation of abietic acid.

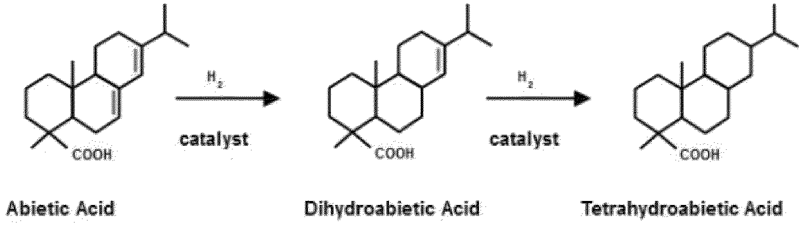

The rosin acid derivative may be at least partially hydrogenated, preferably fully hydrogenated. The rosin acid derivative may be both hydrogenated and esterified. For example, the rosin may be a hydrogenated glyceryl ester.

The rosin acid derivative may be a rosin acid dimer. Dimerization may be useful for increasing the softening point and/or stability of a plant rosin acid. A sample dimerization reaction of abietic acid is shown below.

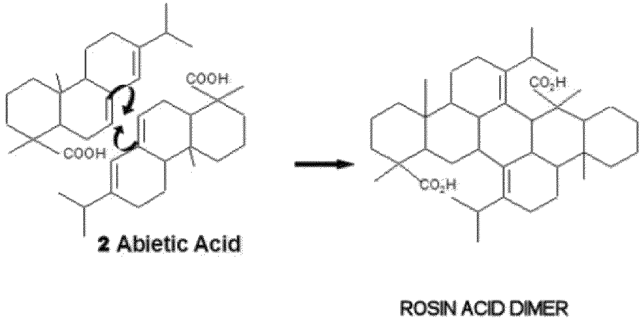

As it is difficult or even impossible to completely dimerize a sample of plant rosin acid, rosin acid dimers are often present with undimerized plant rosin acids. Rosin acid dimers may be further esterified.

A rosin derivative may be dimerized through ions such as Zi2+ or Ca2+. For example, zinc rosinates are rosin derivatives where two abietic acid compounds are bound to a zinc ion.

The rosin derivative may be a rosin acid polymer. As used here, said polymer may comprise three or more monomeric units derived from plant rosin acids. The polymer may be a main-chain polymer or a side-chain polymer.

The rosin derivative may be a rosin acid soap, where a plant rosin acid is reacted with an alkali metal hydroxide (e.g., NaOH or KOH) or an alkaline earth metal hydroxide (e.g., Ca(OH)2). More broadly, the rosin derivative may be a rosin acid salt.

The rosin derivative may be a functionalized rosin acid. In other word, the rosin may be functionalized, where one or more functional groups are added to the plant rosin acid.

A rosin derivative may include the product of a Diels-Alder reaction, such as the reaction product of a plant rosin acid and maleic anhydride; such reaction products may be polymerized.

A rosin derivative may include phenolic rosin acids, where a plant rosin acid is reacted with a phenol.

A rosin derivative may include a rosin alcohol, wherein one or more of the carboxyl groups of the plant rosin acid are converted to hydroxyl groups.

Commercially available rosin derivatives that are suitable for the presently disclosed compositions and methods may include those disclosed in Example 1 of the Examples section below.

The rosin is a plant rosin acid, a rosin acid derivative, or a mixture thereof.

Preferably, the rosin is a plant rosin acid, a rosin acid ester, or a mixture thereof.

Plant rosin acids may be selected from abietic acids, pimaric acids, plicatic acid, or mixtures thereof, preferably abietic acids.

Plant rosin acids may be selected from abietic acid, neoabietic acid, dehydroabietic acid, palustric acid, levopimaric acid, pimaric acid, isopimaric acid, sandaracopimaric acid, plicatic acid, gum plant rosin acids, wood plant rosin acids, tall oil plant rosin acids or mixtures thereof.

Rosin acid derivatives may be selected from rosin esters, abietic rosin esters, pimaric rosin esters, hydrogenated rosin acids, hydrogenated abietic rosin esters, polymerized rosin acids, rosin acid dimers, zinc rosinates, rosin acid polymers, rosin acid soaps, rosin acid salts, functionalized rosin acids, phenolic rosin acids, rosin alcohols, or mixtures therof.

The rosin may be selected from abietic acid, neoabietic acid, dehydroabietic acid, palustric acid, levopimaric acid, pimaric acid, isopimaric acid, sandaracopimaric acid, plicatic acid, gum plant rosin acids, wood plant rosin acids, tall oil plant rosin acids, rosin esters, abietic rosin esters, pimaric rosin esters, hydrogenated rosin acids, hydrogenated abietic rosin esters, polymerized rosin acids, rosin acid dimers, zinc rosinates, rosin acid polymers, rosin acid soaps, rosin acid salts, functionalized rosin acids, phenolic rosin acids, rosin alcohols, or mixtures therof, preferably rosin esters.

In a preferred aspect of the present invention, the rosin may be a rosin ester, wherein the rosin ester is an ester of at least one plant rosin acid and of an alcohol, wherein the at least one plant rosin acid is an abietic acid.

In another preferred aspect of the present invention, the alcohol may comprise a carbon chain, wherein the carbon chain comprises from one to five carbon atoms, preferably from one to three carbon atoms, wherein the carbon atoms are linked to each other through covalent bonds, wherein the alcohol is selected from methanol, glycerol, pentaerythritol, or mixtures thereof, preferably glycerol, wherein the rosin ester has a softening point of from 70°C to 150°C, preferably from 75°C to 130°C, more preferably from 78°C to 110°C, most preferably from 80°C to 90°C.

When the alcohol is a glycerol and the rosin ester has a softening point of from 80°C to 90°C, the composition displays even more dispersibility and less stickiness to the walls of the container.

Without wishing to be bound by theory, rosin ester molecules having relatively high molecular weight and high softening point (such as more than 70°C-80°C) are particularly sticky towards the container material, compromising the long-term stability of the composition. These rosins particularly benefit from the non-ionic surfactant to form a stable emulsion, thus providing the technical benefits.

### Benefit agents

The particles of the present disclosure comprise one or more benefit agents. As described above, it is believed that the benefit agents become embedded and/or encapsulated in rosin when the particles are formed. The particle formation can thus lead to improved stability, delivery, and/or performance of the benefit agent on a target surface, such as a fabric or hard surface. For example, such embedding and/or encapsulation of a benefit agent may prevent degradation of the benefit agent and/or undesirable interactions with other components of the liquid consumer product.

The compositions of the present disclosure may include the benefit agent and/or particles containing the benefit at a level at which the benefit agent provides its intended benefit when the composition is used as intended. For example, the benefit agent of the particles may be present at a level of from 0.05% to 10%, or from 0.05% to 5%, or from 0.1% to 4%, by weight of the composition.

The benefit agent may be selected from the group consisting of fragrances, silicone oils, waxes, hydrocarbons, higher fatty acids, essential oils, lipids, skin coolants, vitamins, sunscreens, antioxidants, enzymes, malodor reducing agents, odor-controlling materials, antistatic agents, softening agents, insect and moth repelling agents, colorants, whiteness enhancers, defoamers, anti-foaming agents, UV protection agents for fabrics and skin, sun fade inhibitors, anti-allergenic agents, water proofing agents, skin care agents, glycerin, aloe vera, vitamin E, shea butter, cocoa butter, brighteners, antiperspirant actives, emollients, skin sensates, and mixtures thereof.

Alternatively, the benefit agent may be selected from the group consisting of fragrances, waxes, hydrocarbons, essential oils, lipids, skin coolants, vitamins, sunscreens, antioxidants, malodor reducing agents, odor-controlling materials, antistatic agents, insect and moth repelling agents, colorants, whiteness enhancers, defoamers, anti-foaming agents, UV protection agents for fabrics and skin, sun fade inhibitors, anti-allergenic agents, water proofing agents, skin care agents, glycerin, aloe vera, vitamin E, shea butter, cocoa butter, antiperspirant actives, emollients, skin sensates, and mixtures thereof.

Particularly preferred benefit agents for the particles include fragrances. The delivery efficacy of the benefit agent may be most efficacious when the benefit agent is relatively hydrophobic. The benefit agent of the particles comprises fragrances, which may comprise one or more perfume raw materials. The term "perfume raw material" (or "PRM") as used herein refers to compounds having a molecular weight of at least 100 g/mol and which are useful in imparting an odor, fragrance, essence, or scent, either alone or with other perfume raw materials. Typical PRMs comprise inter alia alcohols, ketones, aldehydes, esters, ethers, nitrites, and alkenes, such as terpene. The skilled person is aware of suitable PRMs. Non-limiting examples of suitable PRMs is provided below.

Suitable PRMs may include materials such as geraniol, linalool, linalyl acetate, pyranol, geranyl acetate, anisaldehyde, citral, citronellal, lysmeral, citronellol, rose oxide, tetrahydrolinalool, hydroxycitronellal, betaionone, menthol, cinnamaldehyde, anethole, vanillin, ethyl vanillin, eugenol, cinnamon oil, carvone, piperonal, and mixtures thereof. The perfume raw materials may include naturally derived materials, such as essential oils.

The PRMs may be characterized by their boiling points (B.P.) measured at the normal pressure (760 mm Hg), and their octanol/water partitioning coefficient (P), which may be described in terms of logP, determined according to the test method below. Based on these characteristics, the PRMs may be categorized as Quadrant I, Quadrant II, Quadrant III, or Quadrant IV perfumes, as described in more detail below. A perfume having a variety of PRMs from different quadrants may be desirable, for example, to provide fragrance benefits at different touchpoints during normal usage.

The perfume raw materials may comprise a perfume raw material selected from the group consisting of perfume raw materials having a boiling point (B.P.) lower than 250°C and a LogP lower than 3, perfume raw materials having a B.P. of more than 250°C and a LogP of more than 3, perfume raw materials having a B.P. of more than 250°C and a LogP lower than 3, perfume raw materials having a B.P. lower than 250°C and a LogP more than 3 and mixtures thereof. Perfume raw materials having a boiling point B.P. lower than 250°C and a LogP lower than 3 are known as Quadrant I perfume raw materials. Quadrant 1 perfume raw materials are preferably limited to less than 30% of the fragrance. Perfume raw materials having a B.P. of more than 250°C and a LogP of more than 3 are known as Quadrant IV perfume raw materials, perfume raw materials having a B.P. of more than 250°C and a LogP lower than 3 are known as Quadrant II perfume raw materials, perfume raw materials having a B.P. lower than 250°C and a LogP more than 3 are known as a Quadrant III perfume raw materials.

The fragrance may comprise from 1% to 40%, by weight of the fragrance material, of Quadrant I perfume raw materials, and/or from 60% to 99%, by weight of the fragrance material, of non-Quadrant I perfume raw materials.

In particular, the delivery particles of the present disclosure are useful when the benefit agent of the particles contain hydrophobic perfume raw materials. The hydrophobic perfume raw materials may be characterized by a relatively high logP value, for example a logP of more than 3.0, and may include what is described above as Quadrant III PRMs, Quadrant IV PRMs, or mixtures thereof. The benefit agent of the particles may comprise at least 50%, or at least 60%, or at least 70%, or at least 80%, or at least 90%, or 100%, by weight of the benefit agent, of Quadrant III PRMs, Quadrant IV PRMs, or mixtures thereof. Compositions that comprise such levels of Quadrant III and/or IV PRMs as the benefit agent of the particles may be aqueous and comprise at least 60%, or at least 70%, or at least 80%, or at least 90%, or at least 95%, or at least 97%, by weight of the composition, of water, and/or less than 10%, or less than 5%, or less than 3%, surfactant.

Non-limiting examples of Quadrant III PRMs include iso-bomyl acetate, carvacrol, alphacitronellol, paracymene, dihydro myrcenol, geranyl acetate, d-limonene, linalyl acetate, vertenex, and mixtures thereof.

Non-limiting examples of Quadrant IV (or enduring) PRMs include allyl cyclohexane propionate, ambrettolide, amyl benzoate, amyl cinnamate, amyl cinnamic aldehyde, amyl cinnamic aldehyde dimethyl acetal, iso-amyl salicylate, hydroxycitronellal-methyl anthranilate (known as aurantiol^{®}), benzophenone, benzyl salicylate, para-tert-butyl cyclohexyl acetate, isobutyl quinoline, beta-caryophyllene, cadinene, cedrol, cedryl acetate, cedryl formate, cinnamyl cinnamate, cyclohexyl salicylate, cyclamen aldehyde, dihydro isojasmonate, diphenyl methane, diphenyl oxide, dodecalactone, 1-(1,2,3,4,5,6,7,8- octahydro-2,3,8,8-tetramethyl-2- naphthalenyl)-ethanone (known as iso E super^{®}), ethylene brassylate, methyl phenyl glycidate, ethyl undecylenate, 15-hydroxypentadecanoic acid lactone (known as exaltolide^{®}), 1,3,4,6,7,8-hexahydro- 4,6,6,7,8,8-hexamethyl-cyclopenta-gamma-2-benzopyran (known as galaxolide^{®}), geranyl anthranilate, geranyl phenyl acetate, hexadecanolide, hexenyl salicylate, hexyl cinnamic aldehyde, hexyl salicylate, alpha-irone, gamma-ionone, gamma-n-methyl ionone, para-tertiarybutyl- alpha-methyl hydrocinnamic aldehyde (known as lilial^{®}), lilial (p-t-bucinal)^{®}, linalyl benzoate, 2-methoxy naphthalene, methyl dihydrojasmone, musk indanone, musk ketone, musk tibetine, myristicin, oxahexadecanolide-10, oxahexadecanolide-11, patchouli alcohol, 5-acetyl- 1,1 ,2,3,3,6-hexamethylindan (known as phantolide^{®}), phenyl ethyl benzoate, phenylethylphenylacetate, phenyl heptanol, phenyl hexanol, alpha-santalol, delta-undecalactone, gamma-undecalactone, vetiveryl acetate, yara-yara, ylangene, and mixtures thereof.

In particular, the fragrance materials of the present disclosure may include perfume raw materials that are characterized by certain chemical or structural moieties.

In a preferred aspect of the present invention, the fragrance material may comprise one or more perfume raw material, wherein the one or more perfume raw material comprise a moiety selected from the group consisting of a cycloalkane moiety, a cycloalkene moiety, a branched alkyl moiety, and combinations thereof. The cycloalkane moiety comprises a first ring structure having from five to seven carbons, preferably from five to six carbons, that form the first ring structure, wherein the cycloalkene moiety comprises a second ring structure having from five to seven carbons, preferably from five to six carbons, that form the second ring structure, wherein the branched alkyl moiety comprises from one to four carbon atoms, preferably from two to three carbon atoms. Without wishing to be bound by theory, moieties of the indicated structures are likely to provide a better association with the rosin compared to different moieties.

The one or more perfume raw materials may comprise at least two moieties selected from the group consisting of a cycloalkane moiety, a cycloalkene moiety, and a branched alkyl moiety. The one or more perfume raw materials may be present between 25% and 100%, preferably between 50% and 95%, more preferably between 60% and 90%, even more preferably between 70% and 80%, by weight of the fragrance materials.

### Water

The treatment compositions of the present disclosure may comprise water. The treatment compositions according to the present disclosure may comprise between 1% and 99%, or between 10% and 99%, or between 10% and 96%, or between 12% and 90%, or between 20% and 80%, or between 40% and 80%, by weight of the composition, of water.

In particular, liquid compositions such as heavy-duty laundry detergents, liquid fabric conditioners (e.g., softeners or enhancers), and liquid hard surface cleaners may advantageously be formulated with high levels of water, for example to enhance flowability or dispersibility.

When the composition is in the form of a unit dose composition (for example, a liquid composition encapsulated by a water-soluble film), the water may be present at a level of between 1% and 20%, or between 5% and 15%, by weight of the composition. When the composition is in the form of a compact liquid laundry detergent, the water, may be present between 10% and 50%, or between 20% and 40%, by weight of the composition.

### Adjunct ingredient

The treatment compositions of the present disclosure may further include an adjunct ingredient in addition to the particles of the present disclosure. The adjuncts may be suitable for delivering a treatment benefit to a target surface, such as a fabric or other textile. Adjuncts ingredients, as used herein, may also include agents that facilitate chemical or physical stability in the treatment compositions, such as buffers, structurants/thickeners, and/or carriers.

The adjunct ingredient(s) may be present in the composition at levels suitable for the intended use of the composition. Typical usage levels range from as low as 0.001% by weight of composition for adjuncts such as optical brighteners to 50% by weight of composition for builders.

The adjunct ingredient may be mixed with the slurry of the present invention to produce the treatment composition, especially when using the method of making described below.

The adjunct ingredient is to be considered a structurally separate component of the treatment composition from the rosin and/or the benefit agent. This means that the treatment composition comprises a rosin and a benefit agent and an adjunct ingredient, where each of these components is a separate component and together form the structure of the composition, despite the fact that they might have similar functional characteristics.

In a preferred aspect of the present invention, the adjunct ingredient may be selected from an amine, a surfactant system, a water binding agent, a sulfite, fatty acids and/or salts thereof, enzymes, encapsulated benefit agents, soil release polymers, hueing agents, builders, chelating agents, dye transfer inhibiting agents, dispersants, enzyme stabilizers, catalytic materials, bleaching agents, bleach catalysts, bleach activators, polymeric dispersing agents, soil removal/antiredeposition agents, polymeric dispersing agents, polymeric grease cleaning agents, brighteners, suds suppressors, dyes, hueing agents, free perfumes, encapsulated perfumes, a perfume delivery system, structure elasticizing agents, fabric softening agents, carriers, fillers, hydrotropes, organic solvents, anti-microbial agents and/or preservatives, neutralizers and/or pH adjusting agents, processing aids, fillers, rheology modifiers or structurants, opacifiers, pearlescent agents, pigments, anti-corrosion and/or anti-tarnishing agents, and mixtures thereof, preferably wherein the adjunct ingredient comprises a surfactant system, fabric softening agents or combinations thereof, more preferably wherein the surfactant system comprises anionic surfactant, non-ionic surfactant, cationic surfactant, and/or zwitterionic surfactant, and/or more preferably wherein the fabric softening agents comprise a quaternary ammonium compound, silicone compounds, or both, most preferably the adjunct ingredient is a quaternary ammonium compound.

Alternatively, the adjunct ingredient may be selected from an amine, a surfactant system, a water binding agent, a sulfite, soil release polymers, hueing agents, builders, chelating agents, dye transfer inhibiting agents, dispersants, enzyme stabilizers, catalytic materials, bleaching agents, bleach catalysts, bleach activators, polymeric dispersing agents, soil removal/antiredeposition agents, polymeric dispersing agents, polymeric grease cleaning agents, suds suppressors, dyes, hueing agents, structure elasticizing agents, fabric softening agents, carriers, fillers, hydrotropes, organic solvents, anti-microbial agents and/or preservatives, neutralizers and/or pH adjusting agents, processing aids, fillers, rheology modifiers or structurants, opacifiers, pearlescent agents, pigments, anti-corrosion and/or anti-tarnishing agents, and mixtures thereof, preferably wherein the adjunct ingredient comprises a surfactant system, fabric softening agents or combinations thereof, more preferably wherein the surfactant system comprises anionic surfactant, non-ionic surfactant, cationic surfactant, and/or zwitterionic surfactant, and/or more preferably wherein the fabric softening agents comprise a quaternary ammonium compound, silicone compounds, or both, most preferably the adjunct ingredient is a quaternary ammonium compound.

Several of these adjuncts are described in more detail below.

The adjunct material may comprise a surfactant system, conditioning actives, or combinations thereof. Preferably, the surfactant system comprises anionic surfactant, non-ionic surfactant, cationic surfactant, and/or zwitterionic surfactant. Preferably, the fabric softening agents comprise a quaternary ammonium compound, silicone compounds, or both.

Compositions according to the present disclosure may include a surfactant system. The surfactant system may include one or more surfactants. The compositions of the present disclosure may include between 20% and 75%, or between 25% and 70%, or between 30% and 50%, by weight of the composition, of a surfactant system. Compositions of the present disclosure may include less than 20%, or less than 10%, or less than 5%, or less than 3%, by weight of the composition, of a surfactant system.

The surfactant system may include anionic surfactant, non-ionic surfactant, zwitterionic surfactant, cationic surfactant, amphoteric surfactant, or combinations thereof. The surfactant system may include linear alkyl benzene sulfonate, alkyl ethoxylated sulfate, alkyl sulfate, non-ionic surfactant such as ethoxylated alcohol, amine oxide, or mixtures thereof. The surfactants may be, at least in part, derived from natural sources, such as natural feedstock alcohols.

Suitable anionic surfactants may include any conventional anionic surfactant. This may include a sulfate detersive surfactant, for e.g., alkoxylated and/or non-alkoxylated alkyl sulfate materials, and/or sulfonic detersive surfactants, e.g., alkyl benzene sulfonates. The anionic surfactants may be linear, branched, or combinations thereof. Preferred surfactants include linear alkyl benzene sulfonate (LAS), alkyl ethoxylated sulfate (AES) including sodium laureth sulfate (SLES), alkyl sulfates (AS) including sodium lauryl sulfate (SLS), or mixtures thereof. Other suitable anionic surfactants include branched modified alkyl benzene sulfonates (MLAS), methyl ester sulfonates (MES), and/or alkyl ethoxylated carboxylates (AEC). The anionic surfactants may be present in acid form, salt form, or mixtures thereof. The anionic surfactants may be neutralized in part or in whole, for example, by an alkali metal (e.g., sodium) or an amine (e.g., monoethanolamine). In certain treatment compositions, for example, those that include a cationic surfactant such as a conditioning active, it may be desirable to limit the amount of anionic surfactant present; for example, the treatment composition may comprise less than 5%, or less than 3%, or less than 1%, or less than 0.1%, or even 0%, by weight of the treatment composition, of anionic surfactant.

The surfactant system may include non-ionic surfactant. Suitable non-ionic surfactants include alkoxylated alkyl phenols, alkyl phenol condensates, mid-chain branched alcohols, mid-chain branched alkyl alkoxylates, alkylpolysaccharides (e.g., alkylpolyglycosides), polyhydroxy fatty acid amides, ether capped poly(oxyalkylated) alcohol surfactants, and mixtures thereof.

Suitable zwitterionic surfactants may include any conventional zwitterionic surfactant, such as betaines, including alkyl dimethyl betaine and cocodimethyl amidopropyl betaine, C8 to C18 (for example from C12 to C18) amine oxides (e.g., C12-14 dimethyl amine oxide), and/or sulfo and hydroxy betaines, such as N-alkyl-N,N-dimethylammino-1-propane sulfonate where the alkyl group can be C8 to C18, or from C10 to C14. The zwitterionic surfactant may include amine oxide.

The compositions of the present disclosure may include a fabric softening agent, also called conditioning active. Compositions that contain conditioning actives may provide softness, anti-wrinkle, anti-static, conditioning, anti-stretch, color, and/or appearance benefits. Conditioning actives suitable for compositions of the present disclosure may include quaternary ammonium ester compounds, silicones, non-ester quaternary ammonium compounds, amines, fatty esters, sucrose esters, silicones, dispersible polyolefins, polysaccharides, fatty acids, softening or conditioning oils, polymer latexes, or combinations thereof. Preferably, the treatment composition comprises a conditioning active that comprises a quaternary ammonium ester compound, more preferably a quaternary ammonium ester compound in combination with a silicone. The composition may include between 1% and 40%, or between 1% and 30%, or between 5% and 30%, by weight of the composition, of conditioning active.

Liquid treatment compositions according to the present disclosure may include an external structurant. External structurants can provide physical stability to liquid compositions according to the present disclosure, for example by helping to suspend the delivery particles. Structurants, when present, are preferably present in an effective amount that is capable of suspending the particles in the treatment composition. External structurants may include non-polymeric crystalline, hydroxyfunctional structurants and/or polymeric structurants.

Non-polymeric crystalline, hydroxyl functional structurants may comprise a crystallizable glyceride, which may be pre-emulsified to aid dispersion into the final detergent composition. Suitable crystallizable glycerides include hydrogenated castor oil or "HCO" or derivatives thereof, provided that it is capable of crystallizing in the liquid detergent composition.

Polymeric structurants may include naturally derived structurants and/or synthetic structurants. Naturally derived polymeric structurants include: hydroxyethyl cellulose, hydrophobically modified hydroxyethyl cellulose, carboxymethyl cellulose, polysaccharide derivatives and mixtures thereof. Suitable polysaccharide derivatives include: pectine, alginate, arabinogalactan (gum Arabic), carrageenan, gellan gum, xanthan gum, guar gum and mixtures thereof. The structurant may comprise cellulosic fibers, for example in the form of microfibrillated cellulose. Cellulose may be derived from bacterial, wood, or other plants such as fruit or sugar beet.

Synthetic polymeric structurants include: polycarboxylates, polyacrylates, hydrophobically modified ethoxylated urethanes, hydrophobically modified non-ionic polyols and mixtures thereof. The polycarboxylate polymer may be a polyacrylate, polymethacrylate or mixtures thereof. The polyacrylate may be a copolymer of unsaturated mono- or di-carbonic acid and C1-C30 alkyl ester of the (meth)acrylic acid. Such copolymers are available from Lubrizol Corp. under the tradename Carbopol^{®} Aqua 30.

The compositions of the present disclosure may include solvent, preferably organic solvent, such as a non-aminofunctional organic solvent. Suitable organic solvents may include glycerol, ethylene glycol, 1,3 propanediol, 1,2 propanediol, tetramethylene glycol, pentamethylene glycol, hexamethylene glycol, 2,3-butane diol, 1,3 butanediol, diethylene glycol, triethylene glycol, polyethylene glycol, glycerol formal dipropylene glycol, polypropylene glycol, dipropylene glycol n-butyl ether, and mixtures thereof. Organic solvents can provide physical stability benefits, particularly in compact formulations having relatively low water levels. The compositions of the present disclosure may include from 5% to 80%, or 5 from 10% to 50%, by weight of the composition, of organic solvent.

The compositions of the present disclosure may include additional aesthetic agents, such as those selected from dyes, opacifiers, pearlescent agents, or mixtures thereof.

Treatment compositions according to the present disclosure may include a perfume delivery system. Suitable perfume delivery systems may include core-shell encapsulates, pro-perfumes (such as amine- and/or silicone-based pro-perfumes), and mixtures thereof. Core-shell encapsulates may comprise a core and a shell surrounding the core. The core may comprise a benefit agent such as perfume, and optionally a partitioning modifier such as isopropyl myristate. The shell may comprise an inorganic material, a polymer, for example melamine formaldehyde, polyurea, polyvinyl alcohol, polyacrylate, or a polysaccharide. Suitable encapsulates may be characterized by a volume-weighted median particle size of from 10 microns to 100 microns, or from 10 microns to 50 microns, or from 15 microns to 40 microns. Perfume delivery systems may provide benefits such as improved perfume stability, deposition, and/or longevity, and may be particularly useful for perfume raw materials that do not associate well with the rosins of the present disclosure.

### Consumer product

A second aspect of the present invention relates to a consumer product comprising a bottle, wherein the treatment composition according to the first aspect is contained in the bottle.

The consumer product comprises a bottle. The bottle contains the treatment composition according to the present invention. The bottle may have a circular, oval or polygonal cross-section (e.g. rectangle, square, octagonal, etc.), or some combination of those cross-sections (e.g., a rectangular cross-section with outwardly bowed walls). The bottle, or at least a portion thereof, may be free of sharp angles to further reduce a risk of leakage.

The bottle may comprise a dimensionally stable shell constituting an external layer of the bottle. The dimensionally stable shell can be formed from compressed pulp. A surrounding layer may be attached outside of the shell, such as a label containing user operating instructions or legal indications pertaining to the use or content of the consumer product.

The bottle may further comprise a layer of polymeric material coating an inner surface of the shell, the polymeric material may also be disposed on a portion of the exterior surface of the shell. However, the polymeric material may be limited to only the inner surface of the shell (i.e., the inside of bottle). The layer of polymeric material may delimit a cavity which is occupied at least in part by a treatment composition.

The cavity may have a volume comprised between 200 ml and 3000 ml, or between 300 ml and 2000 ml, or between 400ml and 1500ml, or 450ml and 1000ml, or between 500 and 750ml. The bottle may have a height comprised between 5 cm and 50 cm. The bottle may be at least three times taller than wide. The thickness of the layer of polymeric material may be of less than 0.3 mm and preferably less than 0.1 mm, or less than 0.05 mm, or less than 0.01 mm. The thickness of the pulp-based shell may be comprised between 0.2 mm and 2.0 mm, more preferably between 0.2 mm and 1.0 mm.

The weight ratio of compressed pulp to polymeric material may be at least 50:50, more preferably at least 70:30, more preferably at least 80:20, more preferably at least 90:10, more preferably at least 95:5. In this context a weight ratio of "at least X:Y" should be understood as encompassing any value of ratio X1:Y1 where X1≥X and where Y1≤Y. These ratios are beneficial for the bottle to be processed according to various recycling streams. The polymeric material may comprise a polyolefin or semi-crystalline polymeric material (see details below), which is applied to the inner surface of the shell as a fluid or powder.

The polymeric material can optimally fulfil the function of the barrier between the treatment compositions detailed herein and the fiber material and the environment. The shell and the layer of polymeric material therefore act as a composite two-layer material in which the individual layers, fulfill different tasks in order to obtain a fluid-tight and mechanically stable bottle.

The bottle may be closed with any closure known in the art, for example, a screw cap having an internal thread engaging an external thread of the neck of the bottle. Alternative closure can be provided to the bottle, such as snapped-in cap, rubber lid, cork, etc. An external thread of the bottle can be coated by the polymeric material for providing additional mechanical stability and wear resistance to the screwing/unscrewing operations. Again, in other examples the polymeric material may be limited to only the inner surface of the shell (i.e., the inside of bottle.

The bottle may be provided with any shape and number of ribs for stability purposes. The ribs may be integrally formed with the fiber-based shell. The ribs may be located along the bottom of the bottle and/or sides of the bottle.

The polymeric material used for coating the inner surface of the shell is not limited. For example, the polymeric material may comprise a thermoplastic polymer selected from polyolefins, e.g. polyethylene (incl. PET, LDPE or HDPE), or polypropylene and copolymers thereof, polyamides and polyesters, and copolymers thereof. The polymeric material may also comprise water soluble synthetic polymers, such as polyvinyl alcohol or polysaccharides, such as cellulose. The polymeric material may be coated from a powder, where the powder particles have an average size in the range of 1 to 200 µm, preferably 5 to 100 µm, more preferably 10 to 50 µm. These ranges allow for an even coating, and furthermore allow for charging of the polymeric powder particles to be accomplished by the spraying device.

In a further preferred aspect, the polymeric material may have a bio-based polymer (e.g., polyolefin) content of at least 80% by weight, wherein the bio-base is defined according to the standards ASTM D 6866, CEN/TS 16137 and ISO 16620. This makes it possible to provide a fully bio-based bottle since the pulp-based shell is also bio-based.

The polymeric material may be biodegradable according to the standard DIN EN 13432. This has the advantage that biodegradable polymers can be used for the nucleation-induced crystal growth, which enables good barrier properties.

In addition, it is preferred if the polymeric material is modified with a plasma coating to improve the barrier properties of the bottle. The additional plasma coating is preferably a glass coating based on hexamethyldisiloxane (HMDSO) or a "diamond-like carbon" coating based on acetylene.

Conveniently, the bio-based polymer can be high density polyethylene (HDPE), low density polyethylene (LDPE), polyethylene terephthalate (PET), polyethylene furanoate (PEF), polyethylene isosorbide terephthalate (PEIT), polylactide (PLA), polybutylene succinate (PBS), poly-s-caprolactone (PCL) or polyhydroxyalkanoate (PHA), in particular polyhydroxybutyrate (PHB). These polymers can form spherulites under suitable processing conditions. It is preferred if the bio-based polymer is partly produced from CO2 exhaust gases. This makes the bottle particularly sustainable and CO2 levels can be reduced. It is preferred if the bio-based polymer is made from biomass. This biomass can be wood, algae, wastewater, agricultural or forestry waste, feces, household organic waste, agricultural products from overproduction or expired food.

The polymer of the coating may contain copolymers. The copolymers make it possible to lower the crystallite melting point, which means that the fiber structures of the pulp-based shell are neither affected nor destroyed by excessively high temperatures. The polymeric material has a Post-Consumer Recycled content of at least 30% by weight.

### Methods of making

A third aspect of the present invention relates to a method of making the treatment composition according to the first aspect, said process comprising the following steps: A) mixing a rosin and one or more benefit agents to create a premix; and B) contacting the premix with a water solution comprising a non-ionic surfactant to form an encapsulated perfume slurry; and C) using the slurry to produce the treatment composition.

It was surprisingly found that the process according to the present invention increase the processability of rosin-based treatment compositions. This specific order of addition of the ingredients results in a composition with increased flowability, i.e. less caking and clumping, in microfluidics (silicone tubes), especially with a weight ratio between rosin and benefit agent close to 1:3. Moreover, a reduced buildup of rosin is observed on stainless steel, particularly when the weight ratio between rosin and benefit agent is close to 1:1.

Without wishing to be bound by theory, the specific order of addition of the ingredients, coupled with the use of the non-ionic surfactant, improve the flowability on silicone and stainless steel materials, potentially leading to improved manufacturing, handling and storage of the composition.

The process of making a treatment composition may comprise the step of combining the ingredients (e.g., a rosin, one or more benefit agents, a non-ionic surfactant and an adjunct material) as described herein.

The process of making a treatment composition according to the present disclosure comprise the steps of combining the rosin and the one or more benefit agents to form a premix.

The premix may comprise from 1% to 99%, by weight of the premix, of the rosin. The premix may comprise from 1% to 99%, by weight of the premix, of the benefit agent. The premix may comprise the rosin and the fragrance in a weight ratio of 1:4 to 4:1, preferably 1:3.5 to 2:1, more preferably 1:3.5 to 1:1, most preferably 1:3. It is believed that the stickiness benefit increases being closer to a weight ratio of 1:3.

The rosin may be heated to a temperature equal to or more than the softening point of the rosin. The premix may be made by combining the heated rosin with the benefit agent, and mixing. In order to favor the homogeneity of the premix, the mixing may take place in a heated oil bath set at a temperature equal to the softening point of the rosin. As the samples become homogenous, the temperature can be progressively reduced, which helps to lower the risk of loss of volatile materials (e.g., evaporation of volatile PRMs). As an additional or alternative step to heating, the rosin may be grinded to small granules and mixed with the benefit agent.

By mixing the rosin with the benefit agent, it is believed that a phenomenon of self-encapsulation occurs where the rosin and the benefit agent form the particles according to the present invention which are contained in the premix.

To make the premix composition, the rosin may be provided as small granules to facilitate homogenization of the premix. If the rosin is provided as solids that are larger than desired, the process may include grinding or pulverizing the rosin into smaller granules. In a bench top operation, this may be accomplished with a mortar and pestle.

The premix may comprise an emulsifying agent. The premix may comprise from 1% to 95 %, or from 5% to 95%, preferably from 5 % to 40 % by weight of the premix, of the emulsifying agent. The premix may comprise the rosin and the emulsifying agent in a weight ratio of from 5:95 to 95:5. The premix may comprise the benefit agent and the emulsifying agent in a weight ratio of from 5:95 to 95:5. Suitable emulsifying agents may include surfactants, amphiphilic polymers, or mixtures thereof.

Suitable surfactants may include non-ionic surfactants, anionic surfactants, or mixtures thereof.

Suitable amphiphilic polymers may include graft copolymers, such as poly(ethylene glycol)-poly(vinyl acetate) graft copolymer, polyvinyl caprolactam-polyvinyl acetate-polyethylene glycol graft copolymer, or mixtures thereof. Commercially available graft copolymers may include Sokalon^{®} HP 22 or Soluplus^{®}, both available from BASF.

In a preferred aspect of the present invention, the rosin is grinded into pellets. The rosin may be mixed with the fragrance to produce the premix. The mixing may be performed by stirring with a magnetic bar. The mixture of the premix composition and the base composition may need to be mixed at relatively high shear to ensure suitable incorporation / homogenization of the premix into the base composition.

The rosin may be provided at room temperature (e.g., 20-22°C). The mixture of rosin and the one or more benefit agents may be heated prior to mixing, which can improve ease of homogenization. The mixture may be heated to a temperature up to the softening point of the rosin, preferably to less than the softening point of the rosin, more preferably to a temperature of from 20°C to 60°C, more preferably to a temperature of from 40°C to 50°C. It is preferred that the temperature is not too high, so as to avoid evaporation of volatile benefit agents, particularly fragrance material. Once made, the premix may be stored at ambient temperatures.

The non-ionic surfactant, e.g. a C12-14 EO7, may be solubilized in a water solution. The skilled person will be aware of how to prepare a water solution comprising the non-ionic surfactant. The premix may be mixed with the water solution comprising the non-ionic surfactant to form a slurry. Preferably, the mixing may be performed by vortexing. The mixture may be heated to a temperature up to the softening point of the rosin, preferably to less than the softening point of the rosin, more preferably to a temperature of from 20°C to 60°C, more preferably to a temperature of from 40°C to 50°C. It is believed that gentle heating of the rosin, e.g. from 20°C to 30°C, might be sufficient for mixing with the fragrance for producing a fully homogenized premix. Without wishing to be bound by theory, the emulsifying effect of the non-ionic surfactants according to the present invention permits a rapid homogenization of the slurry even at relatively low temperatures.

The mixing at relatively elevated temperature may occur for variable period of time, from 30 seconds to 4 hours, or even from 30 seconds to 2 hours, or even from 30 seconds to 1 hours, or even from 30 seconds to 30 minutes, or even from 30 seconds to 10 minutes, or even from 30 seconds to 5 minutes, or even from 30 seconds to 1 minute. We surprisingly observed the homogenization of the premix after mixing the ingredients by vortexing for 30 seconds to 1 minute. Without wishing to be bound by theory, the emulsifying effect of the non-ionic surfactants according to the present invention permits a rapid homogenization of the slurry even in relatively short times.

After the mixture has been substantially homogenized, the mixture may be cooled to ambient temperature. If the viscosity of the premix at ambient temperature is lower than 6000cP, then the heating step may not be needed.

The slurry may comprise the rosin and the non-ionic surfactant to a weight ratio of 1:4 to 4:1, preferably 1: 1.65 to 2.45: 1, more preferably 1:1 to 1.35:1, most preferably 1.2:1. The slurry may comprise the rosin and the fragrance in a weight ratio of 1:4 to 4:1, preferably 1:3.5 to 2:1, more preferably 1:3.5 to 1:1, most preferably 1:3.

In a preferred aspect of the present invention, the slurry may be mixed with a cationic surfactant to produce the treatment composition, preferably, wherein the cationic surfactant is a quaternary ammonium compound or a salt thereof.

The slurry may be added to an adjunct ingredient to produce the treatment composition according to the first aspect. Preferably, the adjunct ingredient comprises a fabric softening active or a conditioning active, preferably a quaternary ammonium compound.

The treatment composition may be formed by mixing the slurry and the adjunct ingredient by vortexing. Other adjunct materials may be added before, during, or after the addition of the premix composition.

When using the slurry to make a final product composition, the slurry may be heated, for example heated to around 60°C, before being injected in the finished product or otherwise combined with a base composition. This heating step is most likely to be helpful when the premix is characterized by a relatively high rosin:benefit agent (e.g. perfume) weight ratio, such as 1:1. The heating step may not be required.

Without wishing to be bound by theory, this order of addition improves the ease of homogenization of the mixture.

### Slurry

In another aspect, the invention relates to an encapsulated perfume slurry comprising particles, wherein the particles comprise a rosin and one or more benefit agents, wherein the one or more benefit agents comprises a fragrance material, characterized in that the composition comprises a non-ionic surfactant, wherein the non-ionic surfactant comprises an alkyl alkoxylated surfactant, wherein the alkyl alkoxylated surfactant is selected from an alkyl ethoxylated surfactant, an alkyl propoxylated surfactant or combinations thereof, wherein the alkyl alkoxylated surfactant comprises an alkyl chain and an alkoxy chain, wherein the alkyl chain and the alkoxy chain are covalently linked, wherein the alkyl chain is selected from a linear alkyl chain, a branched alkyl chain, a cycloalkyl chain or mixtures thereof, wherein the alkyl chain comprises between 7 and 15 carbon atoms, wherein the alkoxy chain comprises between 4 and 12 alkoxylate groups.

The particles are as described above. The rosin is as described above. The benefit agents are as described above. The fragrance material is as described above. The non-ionic surfactant is as described above.

In a preferred aspect, the non-ionic surfactant may be present between 1.5% and 8.3%, preferably between 1.5% and 8%, more preferably between 1.5% and 6.5%, by weight of the slurry, or the weight ratio between the rosin and the non-ionic surfactant in the slurry may be 1:1.8 to 1.6:1, preferably 1:1.8 to 1:1.15, more preferably 1:1.8 to 1:1.2, or mixtures thereof.

In another preferred aspect, the weight ratio between the rosin and the fragrance material in the slurry may be 1:4 to 4:1, preferably 1:3 to 2:1, more preferably 1:3 to 1:1.

### Methods of treating fabrics

A fourth aspect of the present invention relates to a method of treating fabrics, the method comprising the following steps: A) obtaining a fabric to be treated; and B) contacting the fabric with a wash liquor, the wash liquor comprising the treatment composition according to the first aspect and water.

The process of treating fabrics comprises the step of obtaining a fabric to be treated, contacting the fabric with a wash liquor, comprising treatment composition and water.

The treatment composition may be diluted in water prior, during, or after contacting the surface or article. The fabric may be optionally washed and/or rinsed before and/or after the contacting step. The composition may be applied directly onto a fabric or provided to a dispensing vessel or drum of an automatic laundry machine.

The method of treating a fabric may include the steps of: (a) optionally washing, rinsing and/or drying the fabric; (b) contacting the fabric with the treatment composition as described herein, optionally in the presence of water; (c) optionally washing and/or rinsing the fabric; (d) optionally drying, whether passively and/or via an active method such as a laundry dryer, or (e) combinations thereof. The method may occur during the wash cycle or the rinse cycle, preferably the rinse cycle, of an automatic washing machine. The fabric may be treated by a wash cycle and then followed by one or more rinse cycles.

For purposes of the present disclosure, treatment may include but is not limited to, scrubbing and/or mechanical agitation. The fabric may comprise most any fabric capable of being laundered or treated in normal consumer use conditions.

Wash liquors that comprise the disclosed compositions may have a pH of from about 3 to about 11.5. When diluted, such treatment compositions are typically employed at concentrations of from about 500 ppm to about 15,000 ppm in solution. When the wash solvent is water, the water temperature typically ranges from about 5 °C to about 90 °C and, the water to fabric ratio may be typically from about 1:1 to about 30:1.

The fabric may be contacted with an anionic surfactant, optionally in the presence of water, prior to being contacted with the treatment composition. The fabric may comprise anionic surfactant that is residual from a washing step. The source of the anionic surfactant may be a detergent composition, such as a heavy-duty liquid laundry detergent, a water-soluble pouch comprising a detergent composition, or a powdered laundry detergent. The detergent composition may further comprise suitable detergent adjuncts. For example, the detergent composition may further comprise cellulase enzyme, fatty acids and/or salts thereof, or mixtures thereof.

The treatment composition may be diluted with water, optionally in a vessel such as an automatic washing machine, to make a rinse liquor. The rinse liquor may comprise anionic surfactant and/or cellulase enzymes, which may be residual or carried-over from a wash cycle. The rinse liquor may be removed from the vessel. The fabric may be dried by any suitable process, such as in an automatic dryer, or by line drying.

The water that is part of the wash liquor and/or the rinse liquor may be characterized by a certain hardness. For example, the water may be characterized by having less than 12 gpg, or less than 10 gpg, of hardness. It is believed that lower levels of hardness can lead to better performance compared to processes that occur in higher levels of hardness.

### Method of cleaning

In another aspect, the invention may relate to a method of cleaning fabrics, the method comprising the following steps:
i) obtaining a first fabric to be cleaned, wherein rosin and non-ionic surfactants according to the present invention are present on the first fabric; and
ii) contacting the first fabric with a first wash liquor, the first wash liquor comprising a first detergent composition and water,

The method of cleaning fabrics may further comprise one or both of the following steps:
iii) contacting a second fabric with a second wash liquor, the second wash liquor comprising a treatment composition according to the present invention and water, wherein step iii occurs before step i of the present method.
iv) contacting a second fabric with a third wash liquor, the third wash liquor comprising a second detergent composition and water, wherein step iv occurs before step i or step iii of the present method.

It is believed that fabrics treated with treatment composition according to the present invention are more easily cleanable from deposited rosin by appropriate detergents, as described above.

The rosin may comprise rosins as described above.

The second wash liquor may be obtained by the description described above for the method of treating fabrics.

The skilled person is aware of the composition of suitable first and second detergent compositions. Suitable first and second detergent compositions may comprise surfactants systems as described above.

The first and third wash liquor may be obtained by the non-limiting example described below:
The process of cleaning fabrics comprises the step of obtaining a fabric to be cleaned, contacting the fabric with a wash liquor, comprising detergent composition and water. Typically, the wash liquor has a temperature of above 0°C to 90°C, or to 60°C, or to 40°C, or to 30°C, or to 20°C. The fabric may be contacted to the water prior to, or after, or simultaneous with, contacting the detergent composition with water. Typically, the wash liquor is formed by contacting the detergent composition to water in such an amount so that the concentration of detergent composition in the wash liquor is from 0.2g/l to 20g/l, or from 0.5g/l to 10g/l, or to 5.0g/l. The method of cleaning fabrics can be carried out in a front-loading automatic washing machine, top loading automatic washing machines, including high efficiency automatic washing machines, or suitable hand-wash vessels._Typically, the wash liquor comprises 90 litres or less, or 60 litres or less, or 15 litres or less, or 10 litres or less of water. Typically, 200g or less, or 150g or less, or 100g or less, or 50g or less of detergent composition is contacted to water to form the wash liquor.

### Test Methods

The skilled person is deemed capable of performing the test methods for determining the parameters used to describe the present invention. Non-limiting examples of said test method methods are described below:

### Test Method for Determining the Logarithm of the Octanol/Water Partition Coefficient (logP)

The value of the log of the Octanol/Water Partition Coefficient (logP) is computed for each PRM in the perfume mixture being tested. The logP of an individual PRM is calculated using the Consensus logP Computational Model, version 14.02 (Linux) available from Advanced Chemistry Development Inc. (ACD/Labs) (Toronto, Canada) to provide the unitless 10 logP value. The ACD/Labs' Consensus logP Computational Model is part of the ACD/Labs model suite.

### Softening Point Test Method

If available, the softening point of a rosin as provided by the manufacturer/supplier is to be used. If not available from the manufacturer/supplier, the softening point is determined according to ASTM E28-18, "Standard Test Methods for Softening Point of Resins Derived from Pine Chemicals," using the version approved July 1, 2018, and published July 2018. More specifically, the Reference Method ("Automated Ring and Ball Softening Point Method") provided therein is to be followed. The method is summarized here. As used herein (and as described in ASTM E28-18), the softening point is defined as the temperature at which a disk of the sample held with a horizontal ring (brass shouldered ring; 19.8 mm inner ring diameter, 23.0 outer diameter, as indicated in the ASTM method) is forced downward a distance of 25.4 mm (1 in.) under the weight of a steel ball (9.53mm diameter; mass between 3.45 and 3.55 g) as the sample is heated at 5C/min in a water, glycerin, silicone oil, ethylene glycol/water, or glycerin/water bath.

*Sample Preparation:* Select a representative sample of the rosin to be tested. The sample should include flakes, pastilles, or freshly broken lumps free of oxidized surfaces; avoid inclusion of finely divided material or dust. Melt the sample in a clean bottle; avoid overheating, and avoid incorporating air bubbles into the sample. The time from the beginning of heating to the pouring of the sample should not exceed 15 minutes. Rest the ring, bottom down, on a metal surface; the ring may be preheated. Pour the melted rosin sample into the ring so as to leave an excess upon cooling. After cooling for at least 30 minutes, remove excess material from the periphery and top of the ring.

*Bath Liquid:* The selection of the bath liquid will depend on the softening point ("SP") of the rosin. For SPs between 35C and 80C, use water (distilled or deionized, freshly boiled). For SPs between 80C and 150C, use USP Glycerin. For SPs above 80C, use Silicone Oil (Polydimethylsiloxane - 200 fluid, 50 cSt, from Dow Corning, Midland, MI). For SPs up to 35C, use a 50/50 (v/v) mixture of Ethylene Glycol and Distilled Water; the bath should be cooled 10 to -25C in a precooled freezer or an isopropyl dry-ice bath.

*Test:* Use a suitable automated ring and ball-softening point instrument with control unit; calibrate according to the manufacturer's instructions. Provide a stir bar to a 600 mL beaker and fill with a bath liquid as provided above, depending on the softening point of the rosin. Set up the apparatus, ring, ball, test insert, support pins as recommended by the manufacturer's instructions. Verify that the control unit is set for the correct bath liquid. Heat the bath so that the temperature of the bath liquid is raised uniformly at a rate of 5C/min. The test is complete when then light beam has been interrupted by the falling ball and material. Record the softening point at the temperature displayed on the unit after the test is completed.

### Acid Number Test Method

If available, the acid number of a rosin as provided by the manufacturer/supplier is to be used. If not available from the manufacturer/supplier, the acid value is determined according to ASTM D465-15 (Reapproved 2020), "Standard Test Methods for Acid Number of Pine Chemical Products Including Tall Oil and Other Related Products," as approved June 1, 2020 and published June, 2020. More specifically, the Referee Method ("Potentiometric Method") provided therein is to be followed. The method is summarized here. Provide freshly chipped samples of rosin, which may be further crushed to facilitate weighing and dissolution; pieces with oxidized surfaces, as well as existing rosin dust or powder, should not be used. If a nonhomogenous liquid, place in a closed bottle with a capillary vent or its equivalent, and heat in a hot water bath; the sample may be agitated during heat, and used after homogenous and well stirred. Based on the following table, transfer the proscribed amount of sample to a 400 mL tall form beaker; add the proper amount of solvent I and swirl to dissolve, heating gently if necessary. Add the proper amount of solvent II, if required, and cool to near room temperature. Immerse each electrode of a glass electrode pH meter (calibrated/standardized according to the manufacturer's instructions) in the solution. Stir with a stir bar. Titrate with a standard alkali solution (a 0.5 N or 0.1 N KOH solution), recording the buret and pH meter readings. Sufficient alkali may be added to bring the pH of the solution to 8. Add alkali in 1.0 mL portions until the change in pH per increment added amounts to 0.3 pH unit. Reduct the additions of alkali to 0.1 mL or smaller until the end point has been passed, as indicated by a significant decrease in pH units er 0.1 mL added. Continue the titration with 1.0 mL portions until it becomes apparent that the inflection point has been well defined. Determine the inflection point (point of maximum change in pH per mL of alkali solution) to the nearest 0.05 mL by plotting the pH readings against the milliliters of alkali used. (For more accuracy, the chance in pH per mL may be plotted against the pH; the peak corresponds to the inflection point.)

The inflection point is considered the end point of the titration. The acid number of the sample, expressed as milligrams of KOH per gram of sample is calculated as follows, and may be reported to the nearest whole number: $Acid Number = \left(A\times N\times 56.1\right) / B$ where: A = alkali solution (in mL) required for titration of the specimen; N = normality of the alkali solution, and B = specimen weight (in grams).

### Color Grade Test Method (Gardner Color)

If available, the color grade (Gardner color) of a rosin as provided by the manufacturer/supplier is to be used. If not available from the manufacturer/supplier, the color grade (Gardner color) is determined according to ASTM D6166-12 (Reapproved 2016), "Standard Test Method for Color of Pine Chemicals and Related Products (Instrumental Determination of Gardner Color)," as approved December 1, 2016, and published December, 2016. The method is summarized here. The color of a liquid sample is measured using an instrument, such as a Gardner Color Comparator L, 115V (ex. BYK), capable of measuring transmitted color and reporting in Gardner colors (or, less preferred, in a color system that can be converted to Gardner colors by known methods, such as those disclosed in the ASTM D6166-12). The instrument is calibrated according to the manufacturer's instructions. To prepare the rosin sample for color analysis, a molten sample of the rosin is introduced to a glass cuvet (10-mm path, unless a different path length is specified by the instrument manufacturer). If the sample is solid, it should comprise freshly broken lumps and be free of dust and finely divided material; the solid should be melted (e.g. in 15 minutes or less, in an oven, sand bath, or oil bath), taking care to avoid overheating and introduction of bubbles. After the molten sample is introduced to the glass cuvet, measurements should be taken while still molten. If the material is cloudy, it should be filtered. The glass cuvet is inserted into the instrument, and the color is measured by following the manufacturer's instructions.

### Flash Point Test Method

If available, the flash point of a rosin as provided by the manufacturer/supplier is to be used. If not available from the manufacturer/supplier, the flash point is determined according to ASTM D92-18, "Standard Test Methods for Flash and Fire Points by Cleveland Open Cup Tester," as approved July 1, 2018, and published July, 2018.

### Fabric Treatment Method

When treating fabrics with a composition according to the present disclosure in the experiments below, the following method is followed unless otherwise indicated. For each treatment, a washing machine (ex Miele) is loaded with 3kg of a fabric load. The fabric load comprises 1065g knitted cotton fabric and 1065g polyester-cotton fabrics (50/50). Additionally, the fabric load comprises twenty terry towel tracers, which weigh together 870g. The washing cycle is run at 95°C. Prior to the test treatment, the load is preconditioned twice, each time using the 95°C short cotton cycle with 79g of unperfumed IEC A Base detergent (ex WFK Testgewebe GmbH), followed by two additional 95°C washes without detergent. For the test treatment, the load is washed using a 40°C short cotton cycle, 1200rpm spin speed with 79g IEC A Base detergent, which is added at the start of the wash cycle in the appropriate dispenser. A dosage of 40 ml of the test fabric treatment composition is added in the appropriate dispenser.

### Viscosity Method

Viscosity of a liquid composition is measured using a DV-E viscometer from Brookfield. The spindle is automatically spun at a rate of 60 rpm until a stable value is given in centipoise (cP). Viscosity of the premix comprising rosin, delivery agent and potentially emulsifying agent is measured using a HAAKE MARS from Thermo Scientific using a 60 mm 1° Cone and a gap size of 52 micrometers. The shear viscosity at 20s-1 can be obtained from a logarithmic shear rate sweep from 0.01s-1 to 1200s-1 at 21°C. The viscosity may be expressed as centipoise (cP).

### Hydrophilic Lipophilic Balance (HLB) Determination

If available, the HLB value as provided by the manufacturer/supplier is to be used. The skilled person is aware of the methods to determine the HLB of a compound, and specifically, of a non-ionic surfactant. Non-limiting examples of suitable methods for different types of material are described in Guo et al., Journal of Colloid and Interface Science 298 (2006) 441-450 and in Surfactants and interfacial phenomena-second edition, by Milton J. Rosen, John Wiley & Sons, Inc., New York. Another suitable method for determining HLB of non-ionic surfactant is the Griffin's method which defines HLB as follows: $HLB = 20 ∗ {M}_{h} / M$ where Mₕ is the molecular mass of the hydrophilic portion of the molecule, and M is the molecular mass of the whole molecule, giving a result on a scale of 0 to 20. An HLB value of 0 corresponds to a completely lipophilic/hydrophobic molecule, and a value of 20 corresponds to a completely hydrophilic/lipophobic molecule.

The HLB value can be used to predict the surfactant properties of a molecule:
- < 10 : Lipid-soluble (water-insoluble)
- > 10 : Water-soluble (lipid-insoluble)
- 1 to 3: anti-foaming agent^{[2]}
- 3 to 6: W/O (water in oil) emulsifier
- 7 to 9: wetting and spreading agent^{[2]}
- 13 to 16: detergents^{[2]}
- 8 to 16: O/W (oil in water) emulsifier
- 16 to 18: solubiliser or hydrotrope^{[2]}

### Particle Size Determination

Images of the concentrated slurries and compositions were collected using an inverted DIAPOT 300 microscope (Nikon, Tokyo) connected to a CCD camera with a digital recorder (Nikon digital sight DS-U1). Few microliters of the solutions were placed on a microscope slide, covered with a glass slip and observed in transmitted light. The images were collected using a 20x objective. The outputs obtained after the microscopy analysis are: (1) list of diameters detected; and (2) counts per each diameter size detected.

Therefore, the volume (V) of each particle is calculated with the following equation: $V = \frac{4}{3} π {r}^{3}$ where r is the radius of each detected particle. Finally, the volume-weighted median particle size is calculated (e.g., via a spreadsheet, such those created in Microsoft ExcelTM), assuming that each particle is a sphere.

### Confocal Raman microscopy on liquid formulations

Confocal Raman microscopy was carried out using a Renishaw InVia^{™} Qontor confocal microRaman system equipped with a front illuminated CCD camera and a research-grade Leica DM 2700 microscope. Maps were collected using a 50x objective (WD 8.2 mm, NA 0.50) and a laser operating at 532 nm with a power of 50 mW and a grating of 1800 l/mm. For each sample, several maps were collected in different regions of the surface. The exposure time for each spectrum was 1 s, in the range 200-1900 cm⁻¹ and a step of 2 µm. Spectra were processed with the Renishaw software WiRE^{™}, corrected for cosmic rays, baseline and noise, and then used to obtain maps. The spectra of the pure phases constituting the samples (perfume raw materials, rosins, non-ionic surfactant, treatment composition, cotton and polyester) were also collected. All spectra were collected using a 50x objective, 100 mW, 1 s per spectrum, and 10 accumulations, and used as reference spectra to perform the analysis of the maps with the WiRE^{™} software.

### Confocal Raman microscopy on model fabrics

For the evaluation of the presence of microcapsules on cotton and PE, fabrics were investigated through 2D confocal Raman microscopy, using a Renishaw InVia^{™} Qontor confocal microRaman system equipped with a front illuminated CCD camera and a research-grade Leica DM 2700 microscope. Maps were collected using a 50x objective (WD 8.2 mm, NA 0.50) and a laser operating at 532 nm with a power of 100 mW and a grating of 1800 l/mm. For each sample, several maps were collected in different regions of the surface. The exposure time for each spectrum was 1 s, with 1 accumulation, in the range 200-1900 cm⁻¹ (10% power, step size 5 µm). Spectra were processed with the Renishaw software WiRE^{™}, corrected for cosmic rays, baseline and noise, and then used to obtain maps.

### Examples

The examples provided below are intended to be illustrative in nature and are not intended to be limiting.

### Example 1.

Table 1A shows a variety of commercially available rosins. Additional information is provided where available.

**Table 1A. Commercially available rosins**

| **No.** | **Rosin Type** | **Derivative Type** | **Additives** | **Softening Point (°C)** | **Acid Value (mg KOH / g)** | **TRADE NAME** | **Mfr. *** |
|---|---|---|---|---|---|---|---|
| 1 | Gum Rosin | - | - | 79 | 163 | - | C |
| 2 | Gum Rosin | Glycerol ester | - | 88 | 8 | Permalyn 5095 | A |
| 3 | Gum Rosin | Pentaerythritol ester | - | 125 | 13 | Lurefor 125 | C |
| 4 | Gum Rosin | Pentaerythritol ester | - | 100 | 15 | Permalyn 5110 | A |
| 5 | Gum Rosin | Hydrogenated | - | 70 | 158 | Staybelite Resin-E | A |
| 6 | Misc. | Partially Hydrogenated | - | 75 | 168 | Foralyn E | A |
| | Rosin | | | | | | |
| 7 | Gum Rosin | Partially dimerized | - | 103 | 146 | Poly-Pale | B |
| 8 | Wood | Hydrogenated glycerol ester | - | 84 | 6 | Foral 85 | B |
| | Rosin | | | | | | |
| 9 | Wood | Hydrogenated pentaerythritol ester | - | 99 | 11 | Foral 105 | B |
| | Rosin | | | | | | |
| 10 | Misc. | Dimerized; Zinc resinate | Zinc salt | 160 | 5 | Zincogral Z | B |
| | Rosin | | | | | | |
| 11 | Wood | Pentaerythritol ester | Maleic-Modified | 132 | 140 | Pentalyn- FC | B |
| | Rosin | | | | | | |
| 12 | Gum Rosin | Dimerized | - | 144 | 145 | Dymerex | A |
| 13 | Rosin acids | Dimerized | - | 138 | 154 | Polygral 140 | B |
| 14 | Wood | Hydrogenated | - | 67 | 165 | Foral AX | B |
| | Rosin | | | | | | |
| 15 | Gum Rosin | Glycerol ester | - | 84 | 6.4 | Ester Gum 8L-M | A |
| 16 | Rosin | Polimerized | Maleic-Modified | 130 | 10 | Dertoline MG 130 | B |
| 17 | Rosin | Pentaerythritol ester | - | 128 | 12 | Dertopoline 125 | B |
| 18 | Gum Rosin | Pentaerythritol ester | | 101 | 13 | Permalyn 6110 | A |

| | | | | | | | |
|---|---|---|---|---|---|---|---|
| * Mfr. = Manufacturer, according to the following key: A -Eastman B - DRT C - Luresa Resinas S.L. | | | | | | | |

Table 1B shows a variety of commercially available non-ionic surfactants. Additional information is provided where available.

**Table 1B. Commercially available non-ionic surfactants**

| Commercial name | Molecular structure | HLB |
|---|---|---|
| Exaal 1315 (EXXONMOBIL) | C_{13-15 (70-30%)} EO₁₀, 37% branching | 13.6 |
| Lutensol XL60 (BASF) | C₁₀ guerbet alcohol EO₆ | 12.5 |
| Lutensol TO7 (BASF) | iso-C₁₃ EO₇ | 12 |
| Marlipal 1216/7 (SASOL) | C₁₂₋₁₄ EO₇ | 12.9 |
| Marlipal 26 E9 (SASOL) | C₁₂₋₁₄ EO₉ | 13.9 |
| Neodol 91-6 (SHELL) | C₉₋₁₁ EO₆ | 12.5 |
| Neodol 45-7 NI (SHELL) | C₁₄₋₁₅ EO₇, 18% branching | 12.3 |
| Tergitol 15s-9 (DOW) | sec alcohol ethoxylate (C₁₁₋₁₅) EO₇ | 12.1 |
| BT5 (NIKKOL) | C₁₂₋₁₄ EO₅ | 10.5 |
| BT7 (NIKKOL) | C₁₂₋₁₄ EO₇ | 12 |
| BT9 (NIKKOL) | C₁₂₋₁₄ EO₉ | 13.5 |
| BT12 (NIKKOL) | C₁₂₋₁₄ EO₁₂ | 14.5 |
| BL9 (NIKKOL) | C₁₂ EO₉ | 14.5 |
| EcosurfTM EH 9 (DOW) | Ethyl hexanol EO/PO₉ | 12.5 |

### Example 2

Liquid fabric softener compositions according to Table 2, below, are provided.

**Table 2. Liquid fabric softener compositions**

| Total composition | A | Comparative composition B | C | Comparative composition D |
|---|---|---|---|---|
| Ingredient name | WT% | | | |
| water | 91.678 | 92.118 | 90.318 | 92.118 |
| HEDP monosodium salt solution | 0.030 | 0.030 | 0.030 | 0.030 |
| Formic Acid 85% | 0.045 | 0.045 | 0.045 | 0.045 |
| HCl 32% | 0.020 | 0.020 | 0.020 | 0.020 |
| Quaternary ammonium compound (ester quat) | 5.60 | 5.60 | 5.60 | 5.60 |
| Antifoam Emulsion | 0.0170 | 0.0170 | 0.0170 | 0.0170 |
| Structurant | 0.17 | 0.17 | 0.17 | 0.17 |
| BT7 (NIKKOL) | 0.44 | 0 | 1.80 | |
| Permalyn 5095 | 0.50 | 0.50 | 1.0 | 1.0 |
| Perfume Raw Material | 1.50 | 1.50 | 1.0 | 1.0 |

According to table 2, compositions A-D share a formulation base but have different amounts of non-ionic surfactant, rosin and benefit agent.

Compositions A and C contain a non-ionic surfactant (BT7), 0.44 wt% and 1.8 wt% respectively. Compositions A has a weight ratio of rosin to benefit agent of 1:3, whereas compositions C have a weight ratio of 1:1.

The comparative compositions B and D (hereafter called B and D) do not fall within the scope of the invention and they are merely used as comparative compositions.

### Example 3

Compositions A-D were tested to determine the stickiness of the compositions towards the walls of containers made of different materials. The compositions were stored either in transparent bottles made of PET (Polyethylene Terephthalate) or in transparent glass bottles.

The test in glass bottles was performed as follows. 20 grams of the composition were prepared in 40 mL-glass bottles, sealed, and stored in upright position for two months.

The test in PET bottles was performed as follows: 100 grams of the composition were prepared in 250 mL-PET bottles, sealed, and stored in upright position for three weeks.

After storage, the bottles were inverted and/or shaken. Pictures were collected for each sample (upright position, upside-down position, after manual shaking) and the visual assessment of stickiness was carried out as described above.

The stickiness of the compositions was determined by macroscopic observation of a) insoluble residues left on the walls of the container and b) oil-water phase separation phenomena. Values relative to stickiness were assigned (see Table 3A) from the following scale:
1 - No insoluble residues, no phase separation
2 - No or few insoluble residues, phase separation present but easily reversible by shaking
3 - Several insoluble residues present, reversible phase separation by shaking
4 - Multiple residues present, irreversible phase separation by shaking

**Table 3A. Stickiness to the container's material**

| Container type | A | B | C | D |
|---|---|---|---|---|
| PET bottle | **1** | **2** | **1** | **3** |
| Glass bottle | **1** | **3** | **2** | **3** |

Compositions A and C leave no insoluble residues and show no water-oil phase separation. The addition of the non-ionic surfactant clearly provides a benefit in terms of reducing the visible residues left on the walls of the container. This is valid for both PET and glass containers and across different timepoints of storage. Secondarily, compositions A-D were analyzed by optical microscopy to determine the stability of microcapsules. As microcapsules are expected to be stable during the washing cycle, the compositions were diluted 1:100 in water, mixed for 2 min and monitored with optical microscopy according to the test method described above.

The stability of the microcapsules was determined visually from the microscopy images based on the a) form of the particles and b) presence of residual elements, such as coalesced microcapsules.

Values relative to microcapsules stability were assigned (see Table 3B) from the following scale:
1 - Spherical microcapsules, no residual elements
2 - Semi- spherical microcapsules, few residual elements
3 - Non-spherical microcapsules, several residual elements
4 - Non-spherical microcapsules, abundant residual elements

**Table 3B. Microcapsules stability**

| A | B | C | D |
|---|---|---|---|
| **1** | **3** | **1** | **3** |

Compositions A and C show abundant spherical microcapsules and no residual elements. This proves that the addition of the non-ionic surfactant to the compositions leads to an improved stability of rosin-based microcapsules.

### Example 4

Compositions A-D were tested to determine their processability and flowability on materials commonly found in manufacturing/transportation facilities, such as stainless steel and silicone.

The residue buildup of compositions on stainless steel and silicone was assessed visually.

The residue buildup on stainless steel was assessed using a 8 x 2 cm2 stainless steel foil. The foil was submerged in 40 mL of the investigated composition, left shaking in a VWR standard analog orbital shaker (speed 5) for 24 hours, rinsed with abundant water and air dried.

After visual assessment of residue build-up left on the stainless steel material, values were assigned (see Table 4) from the following scale:
1 - no visible residue, build-up < 0.1%
2 - no visible residue, build-up 0.1 - 1%
3 - little residue, build-up 1-6 %
4 - visible residue, build up > 7%

The residue buildup on a silicone material was assessed using a microfluidics device having silicone channels. The original sample (not diluted) is loaded into a 1 ml syringe. The syringe is loaded on the Nemesys platform (Syringe pump). The syringe pump is set with a flow rate parameter of 0.01 µL/s. The syringe outlet is connected to the PDMS microfluidic chip. The selected channel on the microfluidic chip is analyzed using a confocal microscope. The skilled person is aware of the structure and functioning of commonly-used microfluidics devices.

After visual assessment of the residues left on the silicone material, values were assigned (see Table 4):
1 - flow is continuous, no lumps
2 - flow is hindered by lumps forming after 20 minutes of flowing
3 - flow is hindered by lumps forming after 5 minutes of flowing
4 - flow is blocked by lump within 1 minute of flowing

**Table 4. Processability/flowability of the compositions**

| Material | A | B | C | D |
|---|---|---|---|---|
| Stainless steel | 1 | 1 | **3** | **4** |
| Silicone | **1** | **3** | **1** | **3** |

Compositions A and C show less residues buildup in microfluidics. On the other hand, the major factor impacting processability on stainless steel seems to be the weight ratio of rosin to benefit agent, as compositions A and B left no residues. However, composition C shows a slight improvement compared to Composition D.

Overall, the addition of non-ionic surfactant to compositions reduces the build-up of rosin residues on stainless steel and silicone tubes.

These results highlight the surprising benefits given by non-ionic surfactant in terms of the enhanced processability and flowability of compositions according to the present invention.

### Example 5

Compositions A-D were tested to determine the efficiency of particle deposition on fabrics.

The composition samples were diluted 1:10 in water. White pure cotton was used as model clothes. The fabrics were cut in 2 × 2 cm² pieces and submerged in a water solution with the samples. After 1 h of mixing at VWR^{®} standard analog orbital shaker (speed 6), the fabrics were removed, dried, placed on a glass slide and mapped by means of 2D confocal Raman microscopy.

Based on visual assessment of the signal from the confocal imaging, the level of particle deposition was assigned values (see Table 5) from the following scale:
1 - Abundant
2 - Present
3 - Scarce
4 - Absent

**Table 5. Deposition on fabrics**

| A | B | C | D |
|---|---|---|---|
| 2 | 2 | 2 | 2 |

Compositions A-D show comparable and good deposition efficiencies on cotton fibers. Overall, the particle deposition efficiency of rosin-based compositions is maintained despite the addition of a non-ionic surfactant according to the present invention.

### Example 6

Compositions A-D were tested to determine the degree of removal of deposited rosin on the fabrics. The removal was assessed both on cotton and PE fibers stained with rosin.

The fabrics were treated with compositions according to the procedure described in Example 5. Fabrics having comparable staining pattern were washed by shaking them at VWR standard analog orbital shaker (speed 6) in a water solution with 5% v/v abundant laundry detergent (liquid Dash detergent) for 60 minutes, rinsed with abundant water and dried. Then, the fabrics were mapped through 2D confocal Raman microscopy, to check for the presence of rosin residues.

Based on visual assessment of the signal from the confocal imaging, values were assigned (see Table 6) from the following scale:
1 - Complete
2 - Almost complete
3 - Partial
4 - Null

**Table 6. Removal of deposited rosin**

| Fabric type | A | B | C | D |
|---|---|---|---|---|
| Cotton fibers | **1** | **3** | **1** | **3** |
| Polyester fibers | **1** | **3** | **1** | **3** |

Deposited rosin on both fabric types was more prone to be removed when fabrics were treated with compositions A and C. The addition of non-ionic surfactants to the compositions makes the deposited rosin more prone to be removed when the fabric is washed with an appropriate detergent.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "40 mm."

## Claims

1. A treatment composition comprising particles and an adjunct ingredient,
wherein the particles comprise a rosin and one or more benefit agents,
wherein the one or more benefit agents comprises a fragrance material,
**characterized in that** the composition comprises a non-ionic surfactant,
wherein the non-ionic surfactant comprises an alkyl alkoxylated surfactant,
wherein the alkyl alkoxylated surfactant is selected from an alkyl ethoxylated surfactant, an alkyl propoxylated surfactant or combinations thereof,
wherein the alkyl alkoxylated surfactant comprises an alkyl chain and an alkoxy chain,
wherein the alkyl chain and the alkoxy chain are covalently linked,
wherein the alkyl chain is selected from a linear alkyl chain, a branched alkyl chain, a cycloalkyl chain or mixtures thereof,
wherein the alkyl chain comprises between 7 and 15 carbon atoms,
wherein the alkoxy chain comprises between 4 and 12 alkoxylate groups.

2. A composition according to claim 1, wherein the non-ionic surfactant has a hydrophilic-lipophilic balance between 10 and 15, more preferably between 11.5 and 13.9, or even between more than 12.5 and 13.9.

3. A composition according to claims 1-2, wherein the alkyl alkoxylated surfactant is an alkyl ethoxylated surfactant, wherein the alkoxy chain is an ethoxy chain, wherein the alkoxylate groups are ethoxylate groups.

4. A composition to any preceding claim, wherein the alkyl chain comprises between 12 and 14 carbon atoms, wherein the alkoxy chain comprises between 7 and 9 alkoxylate groups, wherein the alkyl chain is a linear alkyl chain.

5. A composition according to any preceding claim, wherein the non-ionic surfactant is present between 0.4% and 2.5%, preferably between 0.4% and 2.3%, more preferably between 0.4% and 1.8%, by weight of the composition,
Or
wherein the weight ratio between the rosin and the non-ionic surfactant in the composition is 1:1.8 to 1.6:1, preferably 1:1.8 to 1:1.15, more preferably 1:1.8 to 1:1.2, or mixtures thereof.

6. A composition according to any preceding claim, wherein the rosin is a rosin ester,
wherein the rosin ester is an ester of at least one plant rosin acid and of an alcohol,
wherein the at least one plant rosin acid is an abietic acid.

7. A composition according to any preceding claim, wherein the alcohol comprises a carbon chain, wherein the carbon chain comprises from one to five carbon atoms, preferably from one to three carbon atoms,
wherein the carbon atoms are linked to each other through covalent bonds, wherein the alcohol is selected from methanol, glycerol, pentaerythritol, or mixtures thereof, preferably glycerol,
wherein the rosin ester has a softening point of from 70°C to 150°C, preferably from 75°C to 130°C, more preferably from 78°C to 110°C, most preferably from 80°C to 90°C.

8. The composition according to any preceding claim, wherein the particles are **characterized by** a volume-weighted median particle size between 0.5 um and 40 um, preferably between 1 um and 30 um.

9. A composition according to any preceding claim, wherein the weight ratio between the rosin and the fragrance material in the composition is 1:4 to 4: 1, preferably 1:3 to 2:1, more preferably 1:3 to 1:1.

10. A composition according to any preceding claim, wherein the fragrance material comprises one or more perfume raw material, wherein the one or more perfume raw material comprise a moiety selected from the group consisting of a cycloalkane moiety, a cycloalkene moiety, a branched alkyl moiety, and combinations thereof,
wherein the cycloalkane moiety comprises a first ring structure having from five to seven carbons, preferably from five to six carbons, that form the first ring structure,
wherein the cycloalkene moiety comprises a second ring structure having from five to seven carbons, preferably from five to six carbons, that form the second ring structure,
wherein the branched alkyl moiety comprises from one to four carbon atoms,
preferably from two to three carbon atoms.

11. A composition according to any preceding claim, wherein the adjunct ingredient is selected from an amine, a surfactant system, a water binding agent, a sulfite, fatty acids and/or salts thereof, enzymes, encapsulated benefit agents, soil release polymers, hueing agents, builders, chelating agents, dye transfer inhibiting agents, dispersants, enzyme stabilizers, catalytic materials, bleaching agents, bleach catalysts, bleach activators, polymeric dispersing agents, soil removal/antiredeposition agents, polymeric dispersing agents, polymeric grease cleaning agents, brighteners, suds suppressors, dyes, hueing agents, a perfume delivery system, structure elasticizing agents, fabric softening agents, carriers, fillers, hydrotropes, organic solvents, anti-microbial agents and/or preservatives, neutralizers and/or pH adjusting agents, processing aids, fillers, rheology modifiers or structurants, opacifiers, pearlescent agents, pigments, anti-corrosion and/or anti-tarnishing agents, or mixtures thereof,
preferably wherein the adjunct ingredient comprises a surfactant system, fabric softening agents, or combinations thereof, more preferably wherein the surfactant system comprises anionic surfactant, nonionic surfactant, cationic surfactant, and/or zwitterionic surfactant, and/or more preferably wherein the fabric softening agents comprise a quaternary ammonium compound, silicone compounds, or both;
most preferably the adjunct ingredient is a quaternary ammonium compound.

12. A composition according to claims 1-11, wherein the composition may be liquid treatment composition or a solid treatment composition, preferably a liquid treatment composition,
more preferably, wherein the composition is a liquid fabric care treatment composition, liquid hard surface treatment composition, liquid personal care treatment composition, most preferably a liquid fabric care treatment composition.

13. A consumer product comprising a bottle, wherein the treatment composition according to claims 1-12 is contained in the bottle.

14. A method of making the treatment composition according to claims 1-12, said process comprising the following steps:
A) mixing a rosin and one or more benefit agents to create a premix; and
B) contacting the premix with a water solution comprising a non-ionic surfactant to form an encapsulated perfume slurry; and
C) using the slurry to produce the treatment composition.

15. The method of claim 14, wherein the slurry is mixed with a cationic surfactant to produce the treatment composition, preferably, wherein the cationic surfactant is a quaternary ammonium compound or a salt thereof.

16. A method of treating fabrics, the method comprising the following steps:
A) obtaining a fabric to be treated; and
B) contacting the fabric with a wash liquor, the wash liquor comprising the treatment composition of claims 1-12 and water.

17. An encapsulated perfume slurry comprising particles,
wherein the particles comprise a rosin and one or more benefit agents,
wherein the one or more benefit agents comprises a fragrance material,
**characterized in that** the composition comprises a non-ionic surfactant, wherein the non-ionic surfactant comprises an alkyl alkoxylated surfactant, wherein the alkyl alkoxylated surfactant is selected from an alkyl ethoxylated surfactant, an alkyl propoxylated surfactant or combinations thereof,
wherein the alkyl alkoxylated surfactant comprises an alkyl chain and an alkoxy chain,
wherein the alkyl chain and the alkoxy chain are covalently linked,
wherein the alkyl chain is selected from a linear alkyl chain, a branched alkyl chain, a cycloalkyl chain or mixtures thereof,
wherein the alkyl chain comprises between 7 and 15 carbon atoms,
wherein the alkoxy chain comprises between 4 and 12 alkoxylate groups.

18. A slurry of claim 17, wherein the non-ionic surfactant is present between 1.5% and 8.3%, preferably between 1.5% and 8%, more preferably between 1.5% and 6.5%, by weight of the slurry, or
wherein the weight ratio between the rosin and the non-ionic surfactant in the slurry is 1:1.8 to 1.6:1, preferably 1:1.8 to 1:1.15, more preferably 1:1.8 to 1:1.2, or mixtures thereof.

19. A slurry of claims 17-18, wherein the weight ratio between the rosin and the fragrance material in the slurry is 1:4 to 4:1, preferably 1:3 to 2:1, more preferably 1:3 to 1:1.
